(19) Europäisches Patentamt / European Patent Office / Office européen des brevets

(11) **EP 4 036 291 B1**

(12) **EUROPEAN PATENT SPECIFICATION**

(45) Date of publication and mention of the grant of the patent: **30.09.2026 Bulletin 2026/40**

(21) Application number: **20868406.8**

(22) Date of filing: **25.09.2020**

(51) International Patent Classification (IPC): ***D02J 1/22*** (2006.01) ***C07K 14/435*** (2006.01) ***D01F 4/02*** (2006.01) ***D01D 5/06*** (2006.01) ***D01D 10/06*** (2006.01) ***D06M 11/05*** (2006.01) ***D06M 11/84*** (2006.01) ***D02J 13/00*** (2006.01)

(52) Cooperative Patent Classification (CPC): **D01F 4/02; C07K 14/43518; C07K 14/43586; D01D 5/06; D01D 10/06; D02J 1/222; D02J 1/223; D02J 1/228; D02J 1/229; D02J 13/005; D06M 11/05; D06M 11/84;** D06M 2101/12

(86) International application number: **PCT/JP2020/036312**

(87) International publication number: **WO 2021/060481 (01.04.2021 Gazette 2021/13)**

(54) **METHOD FOR MANUFACTURING PROTEIN FIBER AND METHOD FOR MANUFACTURING PROTEIN FIBER FABRIC**

VERFAHREN ZUR HERSTELLUNG EINER PROTEINFASER UND VERFAHREN ZUR HERSTELLUNG EINES PROTEINFASERGEWEBES

PROCÉDÉ DE FABRICATION DE FIBRE PROTÉIQUE ET PROCÉDÉ DE FABRICATION DE TISSU DE FIBRES PROTÉIQUES

(84) Designated Contracting States: **AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2019 JP 2019177842**

(43) Date of publication of application: **03.08.2022 Bulletin 2022/31**


(73) Proprietor: **CRANE INC.**
**Tsuruoka-shi, Yamagata 997-0052 (JP)**

(72) Inventors:
- **ISHII Hideto**
  **Tsuruoka-shi, Yamagata 997-0052 (JP)**
- **IGARASHI Mizuki**
  **Tsuruoka-shi, Yamagata 997-0052 (JP)**
- **ADACHI Tatsuki**
  **Tsuruoka-shi Yamagata 997-0052 (JP)**
- **ABE Yunosuke**
  **Tsuruoka-shi Yamagata 997-0052 (JP)**

(74) Representative: **Handley, Matthew Edward**
**Venner Shipley LLP**
**St James's**
**79 Oxford Street**
**Manchester M1 6EG (GB)**


(56) References cited:

| | |
|---|---|
| **EP-A1- 3 827 682** | **EP-A1- 3 859 076** |
| **EP-A1- 3 922 764** | **WO-A1-2018/164020** |
| **WO-A1-2018/164020** | **WO-A1-2018/164234** |
| **WO-A1-2019/066047** | **WO-A1-2019/066053** |
| **WO-A1-2019/066053** | **JP-A- 2001 064 866** |
| **JP-B1- S 468 480** | |

Remarks:
The complete document including Reference Table(s) and the Sequence Listing(s) can be downloaded from the EPO website


Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).


EP 4 036 291 B1

## Description

### Technical Field

The present invention relates to a method for producing a protein fiber, a method for producing a protein fiber fabric, and a method for shrink-proofing a protein fiber.

### Background Art

Conventionally, protein fibers such as silk and wool are often used as materials for clothing, bedding, and the like by taking advantage of characteristics such as excellent touch feeling and heat retaining property. In addition, spider silk with high toughness having excellent strength and high stretchability has recently attracted attention, and researches for practical use of artificial spider silk having characteristics of the spider silk have been actively conducted. Application of artificial spider silk to not only clothing but also various materials such as industrial materials and medical materials required to have high strength, high toughness, and the like has been studied. Then, unlike synthetic fibers, all of these protein fibers have biodegradability and require less energy in production and processing, and thus demand is expected to increase in accordance with recent increase in environmental conservation awareness.

Some protein fibers shrink due to contact with moisture (for example, immersion in water or hot water, exposure to a high humidity environment, or the like). When such an unexpected length change due to shrinkage of the protein fiber occurs particularly when the protein fiber comes into contact with moisture for the first time after production, there is a concern that various problems may be caused due to the unexpected length change. For example, if an unexpected length change occurs when the protein fiber is exposed to a high humidity environment during storage immediately after production or when a treatment using moisture such as washing and wet heat setting is performed for the first time after production, not only workability and quality may be deteriorated, but also post-processing may be adversely affected.

Under such circumstances, various methods for shrink-proofing a protein fiber have been disclosed. For example, Patent Literature 1 discloses a method for producing a protein fiber, the method including: a stretching step of bringing a protein raw material fiber containing protein into contact with a liquid or vapor to shrink or stretch the protein raw material fiber; and a drying step of drying the protein raw material fiber subjected to the stretching step while controlling the protein raw material fiber to an arbitrary length. Patent Literature 2 discloses a method for producing a protein fiber in which a protein fiber is subjected to heating and allowed to relax and contract during processing.

### Citation List

#### Patent Literature

Patent Literature 1: WO 2018/164020 A
Patent Literature 2: WO 2019/066047 A1

### Summary of Invention

#### Technical Problem

In general in-line (continuous) spinning, the protein fiber is inevitably loaded with tension in the fiber axis direction in order to move the protein fiber from the upstream side to the downstream side. According to the study of the present inventors, it has been found that when shrink-proofing treatment is performed in the middle of in-line spinning, residual stress is generated in the produced protein fiber due to the tension in the fiber axis direction inevitably generated after the shrink-proofing treatment (for example, the tension at the time of winding), and this residual stress causes shrinkage even in the protein fiber subjected to the shrink-proofing treatment when the protein fiber is brought into contact with moisture for the first time after production, and particularly shrinkage is remarkable when the protein fiber is brought into contact with moisture at a high temperature.

In order to more reliably suppress shrinkage at the time of first contact with moisture after production, it is conceivable to subject the protein fiber after in-line spinning to a shrink-proofing treatment without applying tension in the fiber axis direction as much as possible so as not to generate residual stress as much as possible. However, in this case, crimp occurs when the protein fiber comes into contact with moisture. For this reason, the obtained protein fiber has a problem that usable uses are limited.

Therefore, an object of the present invention is to provide a method capable of producing a protein fiber in which

shrinkage at the time of first contact with moisture after production is more reliably suppressed and generation of crimp is also suppressed. Also, an object of the present invention is to provide a method capable of producing a protein fiber fabric in which shrinkage at the time of first contact with moisture after production is more reliably suppressed and generation of crimp is also suppressed.

Solution to Problem

According to claim 1 the present invention discloses a method for producing a protein fiber, the method comprising:

(i) drawing a protein fibril;
(ii) shrinking the protein fibril in a relaxed state after drawing and before being wound;
(iii) winding the protein fibril to obtain a wound protein fibril; and
(iv) shrinking the wound protein fibril in a relaxed state to produce a protein fibre in which moisture-induced shrinkage is reduced.

In the production method described above, the post-shrinking step may be carried out either as a wet shrinking step or as a dry shrinking step.
In the wet shrinking step, the wound protein fibril is brought into contact with a liquid or vapor in a relaxed state so that the wound protein fibril shrinks.
In the dry shrinking step, the wound protein fibril is heated in a relaxed state so as to shrink the wound protein fibril.
In one example of the wet shrinking step, the wound protein fibril may be immersed in a liquid in a relaxed state so that shrinkage of the wound protein fibril occurs.
The liquid or vapor used in the wet shrinking step may have polarity.
For example, the liquid may be water and the vapor may be water vapor.
The protein fibril used in the production method may include a structural protein. In particular embodiments, the structural protein may be modified fibroin. In some cases, the modified fibroin may be modified spider silk fibroin.
The method may further include preparing a fabric using protein fibers produced by the production method described above.

Advantageous Effects of Invention

According to the present invention, it is possible to provide a method capable of producing a protein fiber in which shrinkage at the time of first contact with moisture after production is more reliably suppressed and generation of crimp is also suppressed. According to the present invention, it is also possible to provide a method capable of producing a protein fiber fabric in which shrinkage at the time of first contact with moisture after production is more reliably suppressed and generation of crimp is also suppressed.

Brief Description of Drawings

Fig. 1 is a diagram schematically illustrating an apparatus for producing a protein fibril according to an embodiment.
Fig. 2 is a diagram schematically illustrating an apparatus for producing a protein fibril according to an embodiment.
Fig. 3 is a diagram illustrating speed control means and temperature control means which can be provided in a high temperature heating furnace in Fig. 2.
Fig. 4 is a diagram schematically illustrating an apparatus (post-shrinking device) for producing a protein fiber according to an embodiment.
Fig. 5 is a diagram schematically illustrating an apparatus (post-shrinking device) for producing a protein fiber according to an embodiment.
Fig. 6 is a diagram schematically illustrating an apparatus (post-shrinking device) for producing a protein fiber according to an embodiment. (a) is a schematic diagram of a device for obtaining skein from a wound product of protein fibrils. (b) is a schematic diagram of a wet shrinking device. (c) is a schematic diagram of a dry shrinking device.
Fig. 7 is a schematic diagram illustrating an example of a domain sequence of modified fibroin.
Fig. 8 is a diagram illustrating a distribution of values of z/w (%) in naturally derived fibroin.
Fig. 9 is a diagram illustrating a distribution of values of x/y (%) in naturally derived fibroin.
Fig. 10 is a schematic diagram illustrating an example of a domain sequence of modified fibroin.
Fig. 11 is a schematic diagram illustrating an example of a domain sequence of modified fibroin.

Description of Embodiments

Hereinafter, preferred embodiments of the present invention will be described in detail with reference to the drawings as the case may be, but the present invention is not limited to the following embodiments. In the drawings, the same or corresponding parts are denoted by the same reference numerals, and redundant description will be omitted as appropriate.

[Method for producing protein fiber]

A method for producing a protein fiber according to the present embodiment includes: a pre-shrinking step of shrinking a protein fibril in a relaxed state after drawing and before being wound; a winding step of winding the protein fibril subjected to the pre-shrinking step to obtain a wound product; and a post-shrinking step of shrinking the protein fibril subjected to the winding step in a relaxed state.

The method for producing a protein fiber according to the present embodiment includes the post-shrinking step, so that shrinkage of the obtained protein fiber at the time of first contact with moisture after production is more reliably suppressed. This effect is remarkable particularly when the temperature of the moisture in contact is high. In addition, since the method for producing a protein fiber according to the present embodiment includes the pre-shrinking step in addition to the post-shrinking step, generation of crimp when the obtained protein fiber comes into contact with moisture is sufficiently suppressed.

In the present specification, the phrase "shrinking a protein fibril in a relaxed state" includes not only a case where the protein fibril is shrunk naturally in a relaxed state without loading any tension on the protein fibril, but also a case where the protein fibril is shrunk under a tension loaded to such an extent that the amount of shrinkage of the protein fibril is substantially the same as the amount of shrinkage when the protein fibril is shrunk naturally in a relaxed state without loading any tension on the protein fibril. Note that the phrase "substantially the same" in the present specification refers to the same amount or value, or an amount or value approximate thereto, and the approximate amount or value refers to an amount that can achieve an effect similar to the effect obtained when the same amount or value is used.

[Pre-shrinking step]

The pre-shrinking step is a step of shrinking the protein fibril in a relaxed state after drawing and before being wound. The pre-shrinking step may be performed before the winding step, and may be performed, for example, on a protein fibril spun by a known spinning method (dry spinning, wet spinning, dry-wet spinning, melt spinning, or the like) using a doping liquid (spinning dope) and then drawn by a known drawing method (wet heat drawing, dry heat drawing, or the like).

Examples of the method for shrinking a protein fibril in a relaxed state include a method including a wet shrinking step of bringing a protein fibril into contact with a liquid or vapor in a relaxed state to shrink the protein fibril (wet shrinkage method), a method including a dry shrinking step of heating a protein fibril in a relaxed state to shrink the protein fibril (dry shrinkage method), and the like.

In the wet shrinkage method, the protein fibril shrinks without depending on an external force, by bringing the protein fibril into contact with a liquid or vapor. This is considered to be caused by the liquid or vapor infiltrating between the fibers or into the fiber in the protein fibril having residual stress by drawing to relax the residual stress.

The type of the liquid or vapor used in the wet shrinkage method is not particularly limited as long as the liquid or vapor can naturally shrink the protein fibril by contact with the protein fibril. Examples of the liquid or vapor include a liquid or vapor having polarity. Examples of the liquid having polarity include water, tetrahydrofuran, acetonitrile, acetone, methanol, and the like, and among them, water is suitably used. This is because not only water is inexpensive and excellent in handleability, but also can more quickly and reliably shrink the protein fibril by coming into contact with the protein fibril. Examples of the vapor having polarity include vapor of the above-described liquid having polarity, and among them, water vapor is suitably used. This is because water vapor, like water, can more quickly and reliably shrink the protein fibril by coming into contact with the protein fibril.

In the wet shrinkage method, it is preferable to bring the liquid in a heated state into contact with the protein fibril. Thereby, the shrinkage time of the protein fibril can be further advantageously shortened. From the same viewpoint, the liquid used in the wet shrinkage method is further preferably hot water (heated water) than water.

When the wet shrinkage method is performed using a heated liquid, the temperature of the liquid may be a temperature lower than the temperature at which protein contained in the protein fibril is decomposed or the protein fibril is thermally damaged. However, in consideration of handleability of the liquid, workability of the wet shrinkage method and the like, the upper limit value of the temperature of the liquid is preferably less than the boiling point. Also, a lower limit of the temperature of the liquid is preferably 10°C or higher, more preferably 40°C or higher, and still more preferably 70°C or higher, from the viewpoint of sufficiently obtaining the effect of shortening the shrinkage time. When water is used as the

liquid, an upper limit of the temperature of the water is preferably 90°C or lower and more preferably 80°C or lower. A lower limit of the temperature of the water is preferably 10°C or higher, more preferably 40°C or higher, and still more preferably 70°C or higher. In addition, the temperature of the liquid may be constant or may be varied so as to be a predetermined temperature while the liquid is brought into contact with the protein fibril.

In the wet shrinkage method, a method for bringing the liquid or vapor into contact with the protein fibril is not particularly limited. Examples of the method can include a method of immersing the protein fibril in the liquid, a method of spraying the liquid onto the protein fibril at room temperature or in a heated steam state, and a method of exposing the protein fibril to a high humidity environment filled with vapor. Among these methods, the method of immersing the protein fibril in the liquid is preferable because the shrinkage time can be effectively shortened and the shrinkage processing equipment can be simplified, and the like.

The time for bringing the protein fibril into contact with the liquid is not particularly limited, and may be, for example, 1 minute or longer. The time may be 10 minutes or longer, 20 minutes or longer, or 30 minutes or longer. In addition, an upper limit of the corresponding time is not particularly limited, and may be, for example, 120 minutes or shorter, 90 minutes or shorter, or 60 minutes or shorter, from the viewpoint of shortening the time in the production process and eliminating the possibility of decomposition (hydrolysis and the like) of the protein fibril.

The wet shrinkage method may further include a step of drying the protein fibril (drying step) subsequent to the wet shrinking step.

A drying method in the drying step is not particularly limited, and may be, for example, natural drying or forced drying using drying equipment. A dry temperature is not limited as long as it is a temperature lower than a temperature at which the protein is thermally damaged, and in general, is a temperature in the range of 20 to 150°C, preferably a temperature in the range of 40 to 120°C, or more preferably a temperature in the range of 60 to 100°C. When the temperature is within the above range, the protein fibril can be more quickly and efficiently dried without causing thermal damage of the protein or the like. A dry time is appropriately selected depending on the dry temperature or the like, and for example, a time during which the influence on the quality and physical properties of the woven or knitted fabric due to overdrying of the protein fibril can be eliminated is employed.

The dry shrinkage method includes a dry shrinking step of heating the protein fibril in a relaxed state to shrink it. The dry shrinking step may include a step of heating the protein fibril (heating step) and a step of shrinking the heated protein fibril in a relaxed state (relaxation shrinking step).

Heating of the protein fibril is preferably equal to or higher than a softening temperature of the protein used in the protein fibril. In the specification, the softening temperature of the protein is a temperature at which shrinkage is initiated due to stress relaxation of the protein fibril. In heating relaxation shrinkage at a temperature equal to or higher than the softening temperature of the protein, the protein fibril shrinks to such an extent that it cannot be obtained by simply removing moisture in the protein fibril. The heating temperature is preferably 80°C or higher, more preferably 180°C to 280°C, still more preferably 200°C to 240°C, and even still more preferably 220°C to 240°C. The heating time is preferably 60 seconds or less, more preferably 30 seconds or less, and still more preferably 5 seconds or less, from the viewpoint of elongation of the protein fibril after the heat treatment. It is considered that the length of the heating time does not significantly affect the stress of the protein fibril.

[Winding step]

The winding step is a step of winding the protein fibril subjected to the pre-shrinking step to obtain a wound product. The winding step may be performed immediately after the pre-shrinking step, or may be performed after another step (for example, a drying step of drying the protein fibril) is performed after the pre-shrinking step.

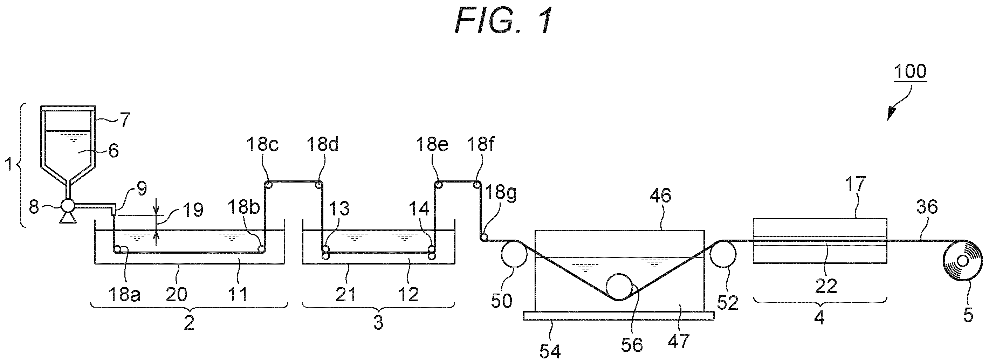

Fig. 1 is a diagram illustrating an example of an apparatus (spinning apparatus) for producing a protein fibril. A spinning apparatus 100 illustrated in Fig. 1 includes an extrusion device 1, a coagulation bath 20, a washing bath 21, a water bath 46 performing a pre-shrinking step, and a drying device 4 in order from an upstream side. Drawing is performed in the coagulation bath 20 and/or the washing bath 21.

The extrusion device 1 includes a storage tank 7, in which a doping liquid (spinning dope) 6 is stored. A coagulation liquid 11 (for example, methanol) is stored in the coagulation bath 20. The doping liquid 6 is pushed out from a nozzle 9 disposed with an air gap 19 between the nozzle 9 and the coagulation liquid 11 by a gear pump 8 attached to a lower end of the storage tank 7. The extruded doping liquid 6 is supplied into the coagulation liquid 11 via the air gap 19. A solvent is removed from the doping liquid 6 in the coagulation liquid 11 to coagulate a protein. The coagulated protein is guided to the washing bath 21, washed by a washing liquid 12 in the washing bath 21, and then sent to the water bath 46 by a first nip roller 13 and a second nip roller 14 disposed in the washing bath 21. At this time, for example, when the rotation speed of the second nip roller 14 is set to be higher than the rotation speed of the first nip roller 13, a protein fibril is drawn at a ratio corresponding to a rotation speed ratio. The protein fibril drawn in the washing liquid 12 is shrunk while passing through the water bath 46 in a relaxed state after leaving the washing bath 21 (pre-shrinking step), subsequently dried while passing through the drying device 4, and then wound up by a winder (winding step). In this way, the protein fibrils are

finally obtained as a wound product 5 wound around the winder. Incidentally, reference numerals 18a to 18g are yarn guides.

Relay rollers 50 and 52 relaying the protein fibril are disposed on the upstream side and the downstream side of the water bath 46. The water bath 46 includes a heater 54, and liquid (hot water) 47 heated by the heater 54 is accommodated in the water bath 46. In addition, in the water bath 46, a presser roller 56 is installed in a state of being immersed in the liquid (hot water) 47. Accordingly, the protein fibril fed from the upstream side runs toward the downstream side while being immersed in the liquid (hot water) 47 in a state of being wound around the presser roller 56 and pressed in the water bath 46. An immersion time of the protein fibril in the hot water 47 is appropriately controlled according to a running speed of the protein fibril.

In the water bath 46, by controlling the feed speed of the protein fibril by the second nip roller 14 and the winding speed of the protein fibril by the winder, the protein fibril is naturally shrunk by contact with the liquid (hot water) 47 in a relaxed state. For example, when a protein fibril that shrinks by about 20% in the fiber axis direction by immersion in the liquid (hot water) 47 in a relaxed state is used, if the feed speed is set to be lower than 80% of the winding speed, the protein fibril can be naturally shrunk in a relaxed state in the liquid (hot water) 47.

The coagulation liquid 11 may be any solution that can be desolvated, and examples thereof include lower alcohols having 1 to 5 carbon atoms such as methanol, ethanol, and 2-propanol, and acetone. The coagulation liquid 11 may appropriately contain water. The temperature of the coagulation liquid 11 is preferably 0 to 30°C. A passing distance of the coagulated protein in the coagulation liquid 11 (substantially, a distance from the thread guide 18a to the thread guide 18b) only needs to have a length capable of efficiently remove a solvent, and is, for example, 200 to 500 mm. The residence time in the coagulation liquid 11 may be, for example, 0.01 to 3 minutes, and is preferably 0.05 to 0.15 minutes. In addition, the drawing (pre-drawing) may be performed in the coagulation liquid 11.

As the drawing carried out when obtaining the protein fibril, for example, wet heat drawing and dry heat drawing can be also adopted in addition to the pre-drawing performed in the coagulation bath 20 and the drawing performed in the washing bath 21 described above.

The wet heat drawing can be performed in warm water, in a solution obtained by adding an organic solvent or the like to warm water, or in heated steam. The temperature may be, for example, 50 to 90°C and preferably 75 to 85°C. In the wet heat drawing, the undrawn yarn (or pre-drawn yarn) can be drawn, for example, by 1 to 10 times and preferably by 2 to 8 times.

The dry heat drawing can be performed using an electric tubular furnace, a dry heat plate, or the like. The temperature may be, for example, 140°C to 270°C, and is preferably 160°C to 230°C. In the dry heat drawing, the undrawn yarn (or pre-drawn yarn) can be drawn, for example, by 0.5 to 8 times and preferably by 1 to 4 times.

The wet heat drawing and the dry heat drawing may be performed independently or in combination, or may be performed in multiple stages. That is, the wet heat drawing and the dry heat drawing can be performed in suitable combination, for example, in a manner in which a first stage drawing is performed by wet heat drawing and a second stage drawing is performed by dry heat drawing or in a manner in which the first stage drawing is performed by wet heat drawing, the second stage drawing is performed by wet heat drawing, and a third stage drawing is performed by dry heat drawing.

A lower limit value of the final draw ratio is preferably any one of more than 1 time, 2 times or more, 3 times or more, 4 times or more, 5 times or more, 6 times or more, 7 times or more, 8 times or more, and 9 times or more with respect to the undrawn yarn (or pre-drawn yarn), and the upper limit value is preferably 40 times or less, 30 times or less, 20 times or less, 15 times or less, 14 times or less, 13 times or less, 12 times or less, 11 times or less, or 10 times or less with respect to the undrawn yarn (or pre-drawn yarn).

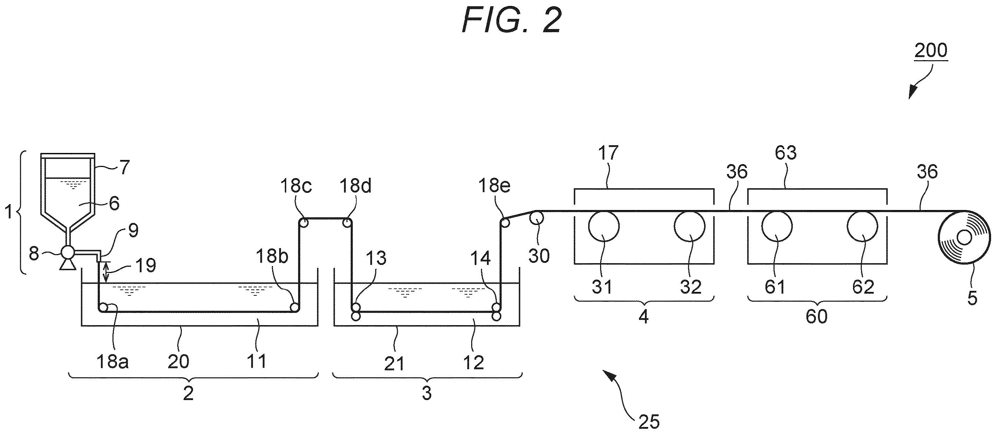

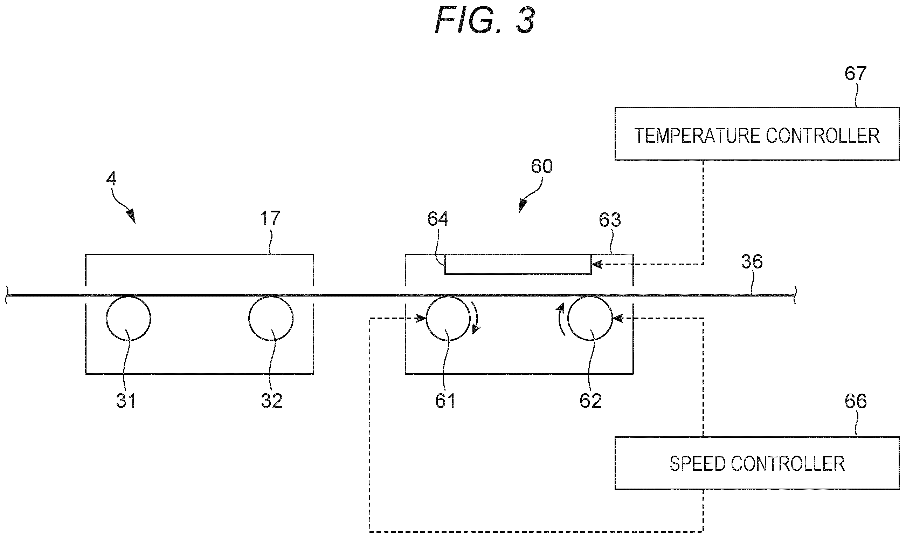

Fig. 2 is a diagram illustrating another example of an apparatus (spinning apparatus) for producing a protein fibril. Fig. 3 is a diagram illustrating speed control means and temperature control means which can be provided in a high temperature heating furnace in Fig. 2. A spinning apparatus 200 illustrated in Fig. 2 is used in a dry shrinkage method, and includes a spinning device (spinning means) 25 for spinning a protein fibril, and a high temperature heating/relaxing device 60 for heating the protein fibril spun by the spinning device 25 in a relaxed state at a high temperature to shrink the protein fibril. In the spinning apparatus 200, a spinning step of spinning the protein fibril and a dry shrinking step of heating the protein fibril in a relaxed state at a high temperature to shrink the protein fibril are continuously performed.

The spinning device 25 is, for example, a spinning device for dry-wet spinning, and includes an extrusion device 1, a coagulation device 2, a washing device 3, and a drying device 4 in order from an upstream side. The extrusion device 1 includes a storage tank 7, and a doping liquid (spinning dope) 6 is stored in the storage tank 7. The coagulation device 2 includes a coagulation bath 20, and a coagulation liquid 11 (for example, methanol) is stored in the coagulation bath 20. The doping liquid 6 is pushed out from a nozzle 9 disposed with an air gap 19 between the nozzle 9 and the coagulation liquid 11 by a gear pump 8 attached to a lower end of the storage tank 7. The extruded doping liquid 6 is supplied into the coagulation liquid 11 via the air gap 19. A solvent is removed from the doping liquid 6 in the coagulation liquid 11 to coagulate a protein.

The washing device 3 includes the washing bath 21, and the washing liquid 12 (for example, water) is stored in the washing bath 21. The protein coagulated in the coagulation bath 20 is guided to the washing bath 21 and washed with the

washing liquid 12. This protein is sent to the drying device 4 by the first nip roller 13 and the second nip roller 14 installed in the washing bath 21. For example, by setting the rotation speed of the second nip roller 14 to be higher than the rotation speed of the first nip roller 13, a protein fibril is drawn at a ratio corresponding to a rotation speed ratio. Incidentally, reference numerals 18a to 18e are yarn guides.

Drawing performed in the washing bath 21 for obtaining the protein fibrils may be so-called wet heat drawing performed in warm water, a solution obtained by adding an organic solvent or the like to warm water, or the like. The temperature in this wet heat drawing may be, for example, 0 to 90°C, is preferably 20 to 70°C, and more preferably 30 to 60°C. The draw ratio of the undrawn yarn (or pre-drawn yarn) in the wet heat drawing may be, for example, 1 to 10 times or 2 to 8 times.

The protein fibrils drawn in the washing liquid 12 are dried while passing through the drying device 4 after leaving the washing bath 21. The drying device 4 includes, for example, a dry heat type drying furnace 17. In the drying furnace 17, a feeding roller 31 and a winding roller 32 are provided. The protein fibril stays in the drying furnace 17 for a predetermined staying time by the feeding roller 31 and the winding roller 32, and then is fed to the high temperature heating/relaxing device 60. In the drying furnace 17, the protein fibril may be drawn at a ratio corresponding to a rotation speed ratio, by setting the rotation speed of the winding roller 32 to be higher than the rotation speed of the feeding roller 31. A heater (not illustrated) is provided in the drying furnace 17. The temperature in the drying furnace 17, that is, the drying temperature of the protein fibril is, for example, 80°C. An oiling device 30 may be provided between the washing device 3 and the drying device 4.

The high temperature heating/relaxing device 60 is provided at the downstream side of the spinning device 25 in the running direction of the protein fibril. The high temperature heating/relaxing device 60 includes, for example, a dry heat type high temperature heating furnace 63. In the high temperature heating furnace 63, a feeding roller (feeding means) 61 and a winding roller (winding means) 62 are provided. The feeding roller 61 and the winding roller 62 both have a cylindrical shape, and the protein fibril is wound around the circumferential surfaces thereof. The protein fibril stays in the high temperature heating furnace 63 for a predetermined staying time by the feeding roller 61 and the winding roller 62, and then is wound up by a winder.

In the high temperature heating furnace 63, for example, by setting the rotation speed of the winding roller 62 to be slower than the rotation speed of the feeding roller 61, the protein fibril is relaxed at a ratio corresponding to a rotation speed ratio. That is, the feeding roller 61 is configured to continuously feed the protein fibril at a predetermined feed speed. The winding roller 62 is configured to continuously wind the protein fibril fed by the feeding roller 61 at a winding speed lower than the feed speed of the feeding roller 61. According to the feeding roller 61 and the winding roller 62 configured as described above, the protein fibril is overfed, and a relaxed state of the protein fibril occurs between the feeding roller 61 and the winding roller 62.

The high temperature heating/relaxing device 60 will be described in more detail with reference to Fig. 3. A speed controller 66 is connected to the feeding roller 61 and the winding roller 62. For example, the speed controller 66 is connected to a drive motor (not illustrated) provided in each of the feeding roller 61 and the winding roller 62. The speed controller 66 is, for example, a computer including hardware such as a CPU, a ROM, and a RAM, and software such as a program stored in the ROM. The speed controller 66 controls both or any one of the feeding roller 61 and the winding roller 62 to control the feed speed and/or the winding speed as described above. That is, the speed controller 66 constitutes a speed control means that controls at least one of the feed speed of the feeding roller 61 and the winding speed of the winding roller 62. For example, the speed controller 66 can control the feed speed and/or the winding speed such that the feed speed by the feeding roller 61 becomes an arbitrary ratio (relaxation ratio) within a range of 1 to 3 times the winding speed by the winding roller 62.

In the high temperature heating furnace 63 of the high temperature heating/relaxing device 60, for example, a high temperature heater 64 is attached. A temperature controller 67 is connected to the high temperature heater 64. The temperature controller 67 is, for example, a computer including hardware such as a CPU, a ROM, and a RAM, and software such as a program stored in the ROM. The temperature controller 67 controls the high temperature heater 64 to control the temperature in the high temperature heating furnace 63. Information on the temperature in the high temperature heating furnace 63 may be input to the temperature controller 67 from a temperature sensor (not illustrated) provided in the high temperature heating furnace 63, and the temperature controller 67 may control the high temperature heater 64 based on the information. The temperature controller 67 constitutes a temperature control means that controls the heating temperature of the protein fibril in the high temperature heating furnace 63. The temperature controller 67 controls the high temperature heater 64 so that the heating temperature in the high temperature heating furnace 63 is higher than the heating temperature in the drying furnace 17 of the drying device 4. The temperature controller 67 can control the heating temperature of the protein fibril, for example, so as to be any temperature within a range of 80 to 300°C.

In the present embodiment, the speed controller 66 and the temperature controller 67 are separately illustrated, but the present invention is not limited to such an aspect. For example, the speed controller 66 and the temperature controller 67 may be incorporated in an integrated controller, or a controller that controls the entire spinning apparatus 200 may be provided with functions corresponding to the speed controller 66 and the temperature controller 67.

The high temperature heating/relaxing device 60 constitutes a heating means for heating the protein fibril and a relaxation/shrinkage means for shrinking the heated protein fibril in a relaxed state. In other words, the high temperature heating/relaxing device 60 serves as both a heating means and a relaxation/shrinkage means. In the dry shrinkage method using the high temperature heating/relaxing device 60, a heating step and a relaxation/shrinkage step are performed simultaneously. As illustrated in Fig. 2, for example, a winder is provided at the downstream side of the high temperature heating/relaxing device 60 in the running direction of the protein fibril. The protein fibril is subjected to a shrinkage treatment in the high temperature heating/relaxing device 60, and then wound by a winder to obtain a wound product 5.

In the relaxation and shrinking step, the relaxation ratio preferably exceeds 1 time, more preferably 1.4 times or more, even still more preferably 1.7 times or more, and particularly preferably 2 times or more. The relaxation ratio is understood as, for example, a ratio of the feed speed to the winding speed of the protein fibril.

[Post-shrinking step]

In the post-shrinking step, the protein fibril subjected to the winding step is shrunk in a relaxed state. By performing the post-shrinking step, shrinkage of the obtained protein fiber at the time of first contact with moisture after production is more reliably suppressed. As shown in Examples described later, it has been found that, in a case where the post-shrinking step is omitted, shrinkage occurs when the protein fiber is brought into contact with moisture for the first time after production, and particularly shrinkage is remarkable when the protein fiber is brought into contact with moisture at a high temperature. This is considered to be caused by generation of residual stress in the protein fibril due to inevitable load of tension in the fiber axis direction to the protein fibril at the time of winding in the winding step. The post-shrinking step is performed for the purpose of alleviating the total residual stress of the protein fibril including the residual stress generated in the winding step.

In the post-shrinking step, examples of the method for shrinking a protein fibril in a relaxed state include a method including a wet shrinking step of bringing a protein fibril into contact with a liquid or vapor in a relaxed state to shrink the protein fibril (wet shrinkage method), and a method including a dry shrinking step of heating a protein fibril in a relaxed state to shrink the protein fibril (dry shrinkage method). Specific aspects of the wet shrinkage method and the dry shrinkage method are as described in the pre-shrinking step.

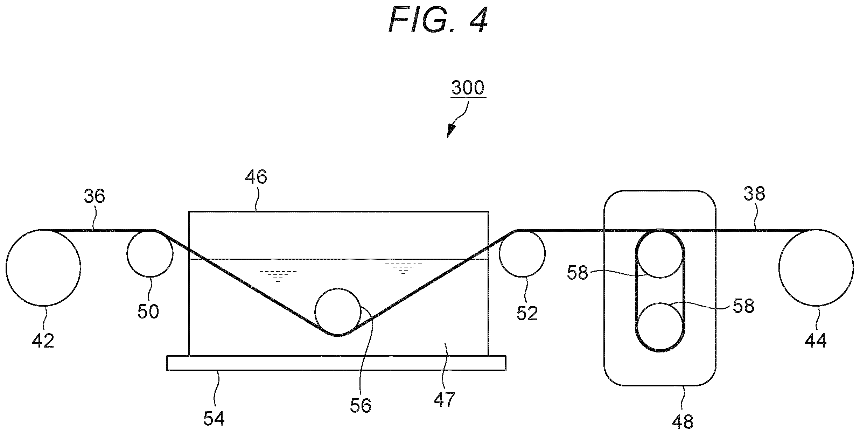

Fig. 4 is a diagram illustrating an example of an apparatus (post-shrinking device) for producing a protein fiber. A post-shrinking device 300 illustrated in Fig. 4 is used in the wet shrinkage method, and includes a feed roller 42 for feeding the protein fibril, a winder 44 for winding a protein fiber, a water bath 46 for performing the post-shrinking step, and a dryer 48 for performing the drying step.

More specifically, the feed roller 42 can be loaded with a wound product of a protein fibril 36, and the protein fibril 36 can be continuously and automatically fed from the wound product of the protein fibril 36 by rotation of an electric motor or the like (not illustrated). The winder 44 can continuously and automatically wind the protein fiber 38 produced through the post-shrinking step and the drying step after being fed out from the feed roller 42 by the rotation of the electric motor (not illustrated). Here, a feed speed of the protein fibril 36 by the feed roller 42 and a winding speed of the protein fiber 38 by the winder 44 can be controlled independently of each other.

The water bath 46 and the dryer 48 are arranged between the feed roller 42 and the winder 44 on the upstream side and the downstream side in a feed direction of the protein fibril 36, respectively. The post-shrinking device 300 illustrated in Fig. 4 includes relay rollers 50 and 52 relaying the protein fibril 36 which runs from the feed roller 42 toward the winder 44.

The water bath 46 includes a heater 54, and liquid (hot water) 47 heated by the heater 54 is accommodated in the water bath 46. In addition, in the water bath 46, a presser roller 56 is installed in a state of being immersed in the liquid (hot water) 47. Accordingly, the protein fibril 36 fed from the feed roller 42 runs toward the winder 44 while being immersed in the liquid (hot water) 47 in a state of being wound around the presser roller 56 and pressed in the water bath 46. An immersion time of the protein fibril 36 in the liquid (hot water) 47 is appropriately controlled according to a running speed of the protein fibril 36.

The dryer 48 has a pair of hot rollers 58. The protein fibril 36 which is released from the water bath 46 and runs toward the winder 44 can be wound around the pair of hot rollers 58. Accordingly, the protein fibril 36 immersed in the liquid (hot water) 47 in the water bath 46 is heated by the pair of hot rollers 58 in the dryer 48, dried, and then further fed toward the winder 44.

When the target protein fiber 38 is produced using the post-shrinking device 300 having such a structure, first, for example, the wound product 5 of the protein fibrils 36 spun using the spinning apparatus 100 illustrated in Fig. 1 is mounted on the feed roller 42. Next, the protein fibril 36 is continuously fed from the feed roller 42 and immersed in the liquid (hot water) 47 in the water bath 46. At this time, by controlling the ratio between the feed speed of the feed roller 42 and the winding speed of the winder 44, the protein fibril is naturally shrunk by contact with the liquid (hot water) 47 in a relaxed state. For example, when a protein fibril that shrinks by about 20% in the fiber axis direction by immersion in the liquid (hot water) 47 in a relaxed state is used, if the feed speed is set to be lower than 80% of the winding speed, the protein fibril can

be naturally shrunk in a relaxed state in the liquid (hot water) 47.

Next, the protein fibril 36 shrunk in the liquid (hot water) 47 in the water bath 46 is heated by a pair of hot rollers 58 of the dryer 48. Thereby, the shrunk protein fibril 36 is dried to form a protein fiber 38. Then, the obtained protein fiber 38 is wound around the winder 44 to obtain the wound product of the protein fiber 38. Instead of the pair of hot rollers 58, the protein fibril 36 may be dried using drying equipment having only a heat source, such as a known dry heat plate.

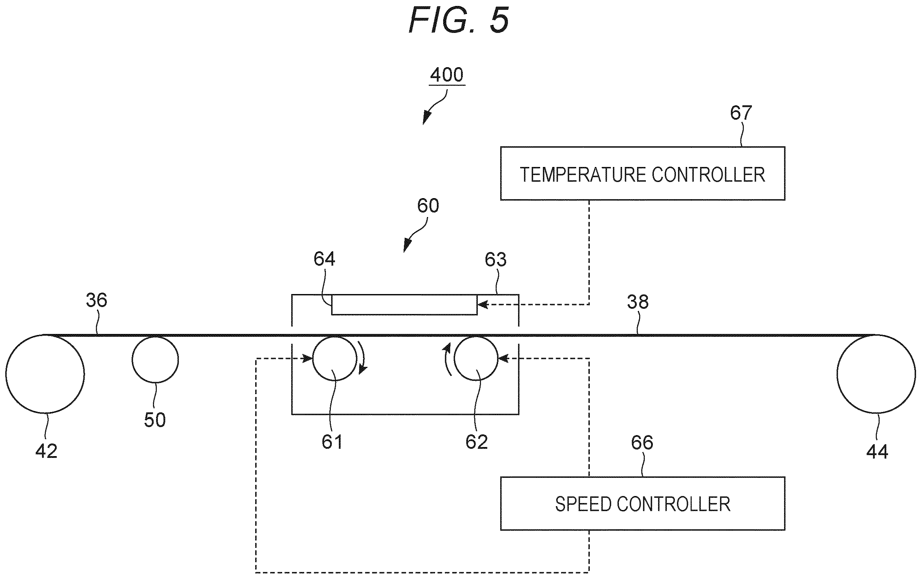

Fig. 5 is a diagram illustrating another example of an apparatus (post-shrinking device) for producing a protein fiber. A post-shrinking device 400 illustrated in Fig. 5 is used in the dry shrinkage method, and includes a feed roller 42 for feeding the protein fibril, a winder 44 for winding a protein fiber, and a high temperature heating/relaxing device 60 for performing the post-shrinking step.

The feed roller 42 can be loaded with a wound product of the protein fibril 36. The high temperature heating/relaxing device 60 is configured as described with reference to Fig. 3. That is, the high temperature heating/relaxing device 60 includes, for example, a dry heat type high temperature heating furnace 63. In the high temperature heating furnace 63, a feeding roller (feeding means) 61 and a winding roller (winding means) 62 are provided. The feeding roller 61 and the winding roller 62 both have a cylindrical shape, and the protein fibril is wound around the circumferential surfaces thereof. The protein fibril stays in the high temperature heating furnace 63 for a predetermined staying time and is shrunk in a relaxed state by the feeding roller 61 and the winding roller 62, and then is wound up by a winder 44.

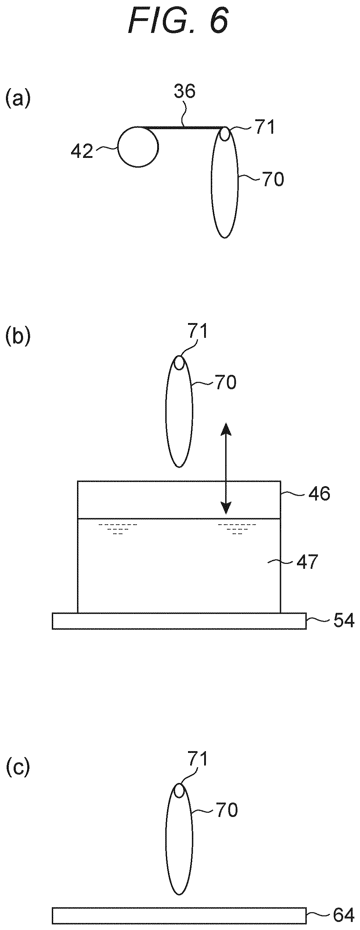

Fig. 6 is a diagram illustrating still another example of an apparatus (post-shrinking device) for producing a protein fiber. Fig. 6(a) is a schematic diagram of a device for obtaining skein from a wound product of protein fibrils. Fig. 6(b) is a schematic diagram of a wet shrinking device. Fig. 6(c) is a schematic diagram of a dry shrinking device.

The post-shrinking device illustrated in Fig. 6 includes a feed roller 42 with which a wound product of the protein fibril 36 can be loaded. First, for example, the wound product 5 of the protein fibril 36 spun using the spinning apparatus 100 illustrated in Fig. 1 is attached to the feed roller 42. Next, the protein fibril 36 is fed out from the feed roller 42 and wound around a support 71 to obtain skein-like protein fibrils 70 (Fig. 6(a)). The skein-like protein fibrils 70 are not under tension and are in a relaxed state.

Then, the skein-like protein fibrils 70 are shrunk in a relaxed state by a wet shrinkage method or a dry shrinkage method. Fig. 6(b) is a schematic diagram of a device used in the wet shrinkage method, and Fig. 6(c) is a schematic diagram of a device used in the dry shrinkage method.

The device illustrated in Fig. 6(b) includes a water bath 46, a heater 54 capable of heating a liquid in the water bath 46, and a liquid (for example, water or hot water) 47 stored in the water bath 46. In the device illustrated in Fig. 6(b), the skein-like protein fibrils 70 are immersed in a heated liquid (for example, hot water) 47. Thereby, the protein fibrils can be shrunk in a relaxed state. Preferred aspects of the liquid (type, temperature, and the like), a preferable aspect of the immersion time, and the like are as described in the pre-shrinking step (wet shrinkage method).

The device illustrated in Fig. 6(c) includes a high temperature heater 64. In the device illustrated in Fig. 6(c), the skein-like protein fibrils 70 are held on the upper part of the high temperature heater 64. As a result, the protein fibrils are heated and shrunk in a relaxed state. The temperature of the high temperature heater 64 is preferably set so that the temperature (heating temperature) of the protein fibrils is equal to or higher than a softening temperature of the protein used in the protein fibril. Preferred aspects of the heating temperature and the heating time and the like are as described in the pre-shrinking step (dry shrinkage method).

[Protein fibril and protein fiber]

The protein fiber produced according to the production method of the present invention or a protein fibril that is an intermediate product contains a protein as a main component. The protein is not particularly limited, and any protein can be used. Examples of the protein can include a natural protein and a recombinant protein (artificial protein). An example of the recombinant protein can include any protein that can be produced in an industrial scale, and examples thereof can include a protein that can be used for industrial purposes, a protein that can be used for medical purposes, and a structural protein. Specific examples of the protein that can be used for industrial purposes or medical purposes can include an enzyme, a regulatory protein, a receptor, a peptide hormone, a cytokine, a membrane or transport protein, an antigen used for vaccination, a vaccine, an antigen-binding protein, an immunostimulatory protein, an allergen, and a full length antibody or an antibody fragment or a derivative thereof. Specific examples of the structural protein can include spider silk, silkworm silk, keratin, collagen, elastin, resilin, and proteins derived from them. As the protein to be used, modified fibroin is preferred, and modified spider silk fibroin is more preferred.

(Modified fibroin)

The modified fibroin according to the present embodiment is a protein containing a domain sequence represented by Formula 1: [(A)n motif-REP]m or Formula 2: [(A)n motif-REP]m- (A)n motif. An amino acid sequence (N-terminal sequence and C-terminal sequence) may be further added to either or both of the N-terminal side and the C-terminal side of

the domain sequence of the modified fibroin. The N-terminal sequence and the C-terminal sequence are not limited thereto, but, typically are regions having no repetitions of amino acid motifs characterized in fibroin, and each consists of amino acids of approximately 100 residues.

The term "modified fibroin" in the present specification refers to artificially produced fibroin (artificial fibroin). The modified fibroin may be fibroin in which a domain sequence is different from an amino acid sequence of naturally derived fibroin or may be fibroin in which a domain sequence is the same as an amino acid sequence of naturally derived fibroin. The term "naturally derived fibroin" as used in the present specification is also a protein containing a domain sequence represented by Formula 1: [(A)n motif-REP]m or Formula 2: [(A)n motif-REP]m- (A)n motif.

The "modified fibroin" may be fibroin obtained by using an amino acid sequence of naturally derived fibroin as it is, fibroin in which an amino acid sequence is modified based on an amino acid sequence of naturally derived fibroin (for example, fibroin in which an amino acid sequence is modified by modifying a cloned gene sequence of naturally derived fibroin), or fibroin artificially designed and synthesized independently of naturally derived fibroin (for example, fibroin having a desired amino acid sequence by chemically synthesizing a nucleic acid encoding a designed amino acid sequence).

The term "domain sequence" in the present specification is an amino acid sequence that produces a crystal region (typically, corresponding to the (A)n motif of the amino acid sequence) and an amorphous region (typically, corresponding to REP of the amino acid sequence) specific to fibroin, and means an amino acid sequence represented by Formula 1: [(A)n motif-REP]m or Formula 2: [(A)n motif-REP]m- (A)n motif. Here, the (A)n motif represents an amino acid sequence mainly consisting of alanine residues, and the number of amino acid residues is 2 to 27. The number of the amino acid residues in the (A)n motif may be an integer of 2 to 20, 4 to 27, 4 to 20, 8 to 20, 10 to 20, 4 to 16, 8 to 16, or 10 to 16. In addition, the proportion of the number of alanine residues in the total number of amino acid residues in the (A)n motif may be 40% or more, or may also be 60% or more, 70% or more, 80% or more, 83% or more, 85% or more, 86% or more, 90% or more, 95% or more, or 100% (meaning that the (A)n motif only consists of alanine residues). At least seven of a plurality of (A)n motifs in the domain sequence may consist of only alanine residues. The REP represents an amino acid sequence consisting of 2 to 200 amino acid residues. The REP may be an amino acid sequence consisting of 10 to 200 amino acid residues. m represents an integer of 2 to 300, and may be an integer of 10 to 300. A plurality of (A)n motifs may be the same amino acid sequences or different amino acid sequences. A plurality of REPs may be the same amino acid sequences or different amino acid sequences.

The modified fibroin according to the present embodiment can be obtained by, for example, performing modification of an amino acid sequence corresponding to substitution, deletion, insertion, and/or addition of one or a plurality of amino acid residues with respect to a cloned gene sequence of naturally derived fibroin. Substitution, deletion, insertion, and/or addition of the amino acid residues can be performed by methods well known to those skilled in the art, such as site-directed mutagenesis. Specifically, the modification may be performed in accordance with a method described in literatures such as Nucleic Acid Res. 10, 6487 (1982), and Methods in Enzymology, 100, 448 (1983).

The naturally derived fibroin is a protein containing a domain sequence represented by Formula 1: [(A)n motif-REP]m or Formula 2: [(A)n motif-REP]m-(A)n motif, and a specific example thereof can include fibroin produced by insects or spiders.

Examples of the fibroin produced by insects can include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama and a hornet silk protein secreted by larvae of Vespa simillima xanthoptera.

More specific examples of the fibroin produced by insects can include the silkworm fibroin L chain (GenBank Accession Nos. M76430 (base sequence) and AAA27840.1 (amino acid sequence)).

Examples of the fibroin produced by spiders can include spider silk proteins produced by spiders belonging to the order *Araneae.* More specific examples thereof can include spider silk proteins produced by spiders belonging to the genus *Araneus,* such as *Araneus ventricosus, Araneus diadematus, Araneus pinguis, Araneus pentagrammicus,* and *Araneus nojimai,* spiders belonging to the genus *Neoscona,* such as *Neoscona scylla, Neoscona nautica, Neoscona adianta,* and *Neoscona scylloides,* spiders belonging to the genus *Pronus,* such as *Pronous* minutus, spiders belonging to the genus *Cyrtarachne,* such as *Cyrtarachne bufo* and *Cyrtarachne inaequalis,* spiders belonging to the genus *Gasteracantha,* such as *Gasteracantha kuhlii* and *Gasteracantha mammosa,* spiders belonging to the genus *Ordgarius,* such as *Ordgarius hobsoni* and *Ordgarius sexspinosus,* spiders belonging to the genus *Argiope,* such as *Argiope amoena, Argiope minuta*, and *Argiope bruennichi,* spiders belonging to the genus *Arachnura,* such as *Arachnura logio,* spiders belonging to the genus *Acusilas,* such as Acusilas coccineus, spiders belonging to the genus *Cytophora,* such as *Cyrtophora moluccensis, Cyrtophora exanthematica,* and *Cyrtophora unicolor,* spiders belonging to the genus *Poltys,* such as *Poltys illepidus,* spiders belonging to the genus *Cyclosa,* such as *Cyclosa octotuberculata, Cyclosa sedeculata, Cyclosa vallata,* and *Cyclosa atrata,* and spiders belonging to the genus *Chorizopes,* such as *Chorizopes nipponicus,* and spider silk proteins produced by spiders belonging to the family *Tetragnathidae,* such as spiders belonging to the genus *Tetragnatha,* such as *Tetragnatha praedonia, Tetragnatha maxillosa, Tetragnatha extensa,* and *Tetragnatha squamata,*

spiders belonging to the genus *Leucauge,* such as *Leucauge magnifica, Leucauge blanda,* and *Leucauge subblanda,* spiders belonging to the genus *Nephila,* such as *Nephila clavata* and *Nephila pilipes,* spiders belonging to the genus *Menosira,* such as *Menosira ornata,* spiders belonging to the genus *Dyschiriognatha,* such as *Dyschiriognatha tenera,* spiders belonging to the genus *Latrodectus,* such as *Latrodectus mactans, Latrodectus hasseltii, Latrodectus geometricus,* and *Latrodectus tredecimguttatus,* and spiders belonging to the genus *Euprosthenops.* Examples of the spider silk protein can include dragline silk proteins such as MaSps (MaSp1 and MaSp2) and ADFs (ADF3 and ADF4), MiSps (MiSp1 and MiSp2), AcSp, PySp, and Flag.

More specific examples of the spider silk protein produced by spiders include fibroin-3 (adf-3) [derived from *Araneus diadematus*] (GenBank Accession No. AAC47010 (amino acid sequence), U47855 (base sequence)), fibroin-4 (adf-4) [derived from *Araneus diadematus*] (GenBank Accession No. AAC47011 (amino acid sequence), U47856 (base sequence)), dragline silk protein spidroin 1 [derived from *Nephila clavipes*] (GenBank Accession No. AAC04504 (amino acid sequence), U37520 (base sequence)), major ampullate spidroin 1 [derived from *Latrodectus hesperus*] (GenBank Accession No. ABR68856 (amino acid sequence), EF595246 (base sequence)), dragline silk protein spidroin 2 [derived from *Nephila clavata*] (GenBank Accession No. AAL32472 (amino acid sequence), AF441245 (base sequence)), major ampullate spidroin 1 [derived from *Euprosthenops australis*] (GenBank Accession No. CAJ00428 (amino acid sequence), AJ973155 (base sequence)), and major ampullate spidroin 2 [*Euprosthenops australis*] (GenBank Accession No. CAM32249.1 (amino acid sequence), AM490169 (base sequence)), minor ampullate silk protein 1 [*Nephila clavipes*] (GenBank Accession No. AAC14589.1 (amino acid sequence)), minor ampullate silk protein 2 [*Nephila clavipes*] (GenBank Accession No. AAC14591.1 (amino acid sequence)), and minor ampullate spidroin-like protein [*Nephilengys cruentata*] (GenBank Accession No. ABR37278.1 (amino acid sequence).

More specific examples of the naturally derived fibroin can include fibroin whose sequence information is registered in NCBI GenBank. For example, sequences thereof may be confirmed by extracting sequences in which spidroin, ampullate, fibroin, "silk and polypeptide", or "silk and protein" is described as a keyword in DEFINITION among sequences containing INV as DIVISION among sequence information registered in NCBI GenBank, sequences in which a specific character string of products is described from CDS, or sequences in which a specific character string is described from SOURCE to TISSUE TYPE.

The modified fibroin according to the present embodiment may be modified silk fibroin (in which an amino acid sequence of a silk protein produced by silkworm is modified), or may be modified spider silk fibroin (in which an amino acid sequence of a spider silk protein produced by spiders is modified).

Specific examples of the modified fibroin can include modified fibroin derived from a major dragline silk protein produced in a major ampullate gland of a spider (first modified fibroin), modified fibroin containing a domain sequence in which the content of glycine residues is reduced (second modified fibroin), modified fibroin containing a domain sequence in which the content of an (A)n motif is reduced (third modified fibroin), modified fibroin in which the content of glycine residues and the content of an (A)n motif are reduced (fourth modified fibroin), modified fibroin containing a domain sequence including a region locally having a high hydropathy index (fifth modified fibroin), and modified fibroin containing a domain sequence in which the content of glutamine residues is reduced (sixth modified fibroin).

An example of the first modified fibroin can include a protein containing a domain sequence represented by Formula 1: [(A)n motif-REP]m. In the first modified fibroin, the number of amino acid residues in the (A)n motif is preferably an integer of 3 to 20, more preferably an integer of 4 to 20, still more preferably an integer of 8 to 20, even still more preferably an integer of 10 to 20, even still more preferably an integer of 4 to 16, particularly preferably an integer of 8 to 16, and most preferably an integer of 10 to 16. In the first modified fibroin, the number of amino acid residues constituting REP in Formula 1 is preferably 10 to 200 residues, more preferably 10 to 150 residues, and still more preferably 20 to 100 residues, and even still more preferably 20 to 75 residues. In the first modified fibroin, the total number of glycine residues, serine residues, and alanine residues included in the amino acid sequence represented by Formula 1: [(A)n motif-REP]m is preferably 40% or more, more preferably 60% or more, and still more preferably 70% or more, relative to the total number of amino acid residues.

The first modified fibroin may be a polypeptide including an amino acid sequence unit represented by Formula 1: [(A)n motif-REP]m, and including a C-terminal sequence which is an amino acid sequence set forth in any one of SEQ ID NO: 1 to 3 or a C-terminal sequence which is an amino acid sequence having 90% or more homology with the amino acid sequence set forth in any one of SEQ ID NO: 1 to 3.

The amino acid sequence set forth in SEQ ID NO: 1 is identical to an amino acid sequence consisting of 50 amino acid residues of the C-terminus of an amino acid sequence of ADF3 (GI: 1263287, NCBI). The amino acid sequence set forth in SEQ ID NO: 2 is identical to an amino acid sequence set forth in SEQ ID NO: 1 in which 20 amino acid residues have been removed from the C-terminus. The amino acid sequence set forth in SEQ ID NO: 3 is identical to an amino acid sequence set forth in SEQ ID NO: 1 in which 29 amino acid residues have been removed from the C-terminus.

A specific example of the first modified fibroin can include modified fibroin including (1-i) the amino acid sequence set forth in SEQ ID NO: 4 (recombinant spider silk protein ADF3KaiLargeNRSH1), or (1-ii) an amino acid sequence having 90% or more sequence identity with the amino acid sequence set forth in SEQ ID NO: 4. The sequence identity is preferably

95% or more.

The amino acid sequence set forth in SEQ ID NO: 4 is obtained by the following mutation: in an amino acid sequence of ADF3 in which an amino acid sequence (SEQ ID NO: 5) consisting of a start codon, a His 10-tag, and an HRV3C protease (Human rhinovirus 3C protease) recognition site is added to the N-terminus, the 1st to 13th repetitive regions are about doubled and the translation ends at the 1154th amino acid residue. The C-terminal amino acid sequence of the amino acid sequence set forth in SEQ ID NO: 4 is identical to the amino acid sequence set forth in SEQ ID NO: 3.

The modified fibroin of (1-i) may consist of the amino acid sequence set forth in SEQ ID NO: 4.

The domain sequence of the second modified fibroin has an amino acid sequence in which the content of glycine residues is reduced, as compared with naturally derived fibroin. It can be said that the second modified fibroin has an amino acid sequence corresponding to an amino acid sequence in which at least one or a plurality of glycine residues in REP are substituted with another amino acid residue, as compared with naturally derived fibroin.

The domain sequence of the second modified fibroin may have an amino acid sequence corresponding to an amino acid sequence in which one glycine residue in at least one or the plurality of motif sequences is substituted with another amino acid residue, in at least one motif sequence selected from GGX and GPGXX (where G represents a glycine residue, P represents a proline residue, and X represents an amino acid residue other than glycine) in REP, as compared with the naturally derived fibroin.

In the second modified fibroin, the proportion of the motif sequence in which the glycine residue has been substituted with another amino acid residue may be 10% or more relative to the entire motif sequence.

The second modified fibroin contains a domain sequence represented by Formula 1: [(A)n motif-REP]m, and may have an amino acid sequence in which z/w is 30% or more, 40% or more, 50% or more, or 50.9% or more in a case where the total number of amino acid residues in the amino acid sequence consisting of XGX (where X represents an amino acid residue other than glycine) included in all REPs in a sequence excluding the sequence from the (A)n motif located at the most C-terminal side to the C-terminus of the domain sequence from the domain sequence is defined as z, and the total number of amino acid residues in the sequence excluding the sequence from the (A)n motif located at the most C-terminal side to the C-terminus of the domain sequence from the domain sequence is defined as w. The number of alanine residues with respect to the total number of amino acid residues in the (A)n motif is 83% or more, preferably 86% or more, more preferably 90% or more, still more preferably 95% or more, and even still more preferably 100% (meaning that the (A)n motif consists of only alanine residues).

The second modified fibroin is preferably one in which the content ratio of the amino acid sequence consisting of XGX is increased by substituting one glycine residue of the GGX motif with another amino acid residue. In the second modified fibroin, the content ratio of the amino acid sequence consisting of GGX in the domain sequence is preferably 30% or less, more preferably 20% or less, still more preferably 10% or less, even still more preferably 6% or less, even still more preferably 4% or less, and particularly preferably 2% or less. The content ratio of the amino acid sequence consisting of GGX in the domain sequence can be calculated by the same method as the calculation method of the content ratio (z/w) of the amino acid sequence consisting of XGX described below.

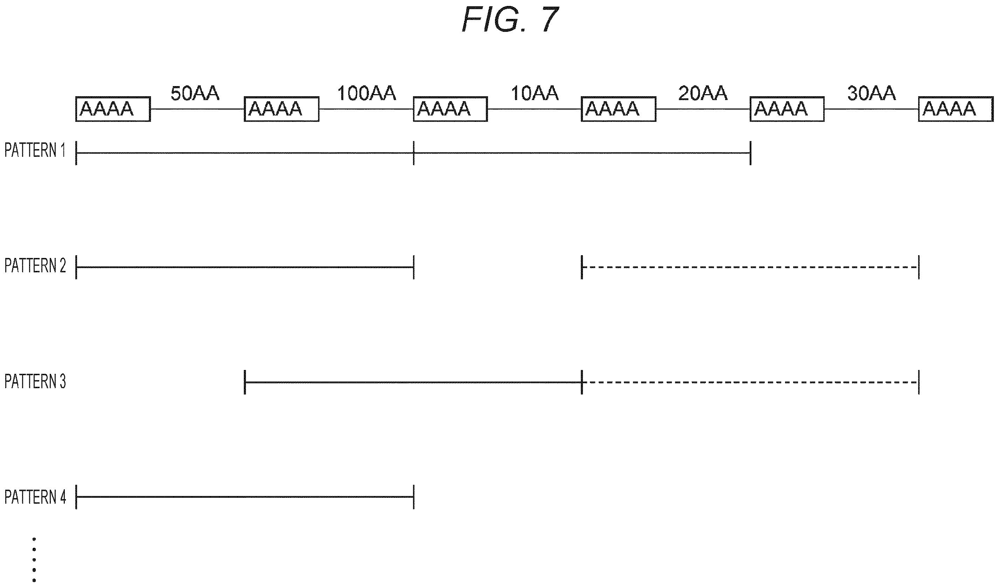

The method of calculating z/w will be described in more detail. First, the amino acid sequence consisting of XGX is extracted from all REPs included in a sequence excluding the sequence from the (A)n motif located at the most C-terminal side to the C-terminus of the domain sequence from the domain sequence in the fibroin containing a domain sequence represented by Formula 1: [(A)n motif-REP]m (modified fibroin or naturally derived fibroin). The total number of amino acid residues constituting XGX is z. For example, in a case where 50 amino acid sequences consisting of XGX are extracted (there is no overlap), z is $50 \times 3 = 150$. Also, for example, in a case where X (central X) included in two XGXs exists as in a case of the amino acid sequence consisting of XGXGX, z is calculated by subtracting the overlapping portion (in a case of XGXGX, it is 5 amino acid residues). w is the total number of amino acid residues included in a sequence excluding the sequence from the (A)n motif located at the most C-terminal side to the C-terminus of the domain sequence from the domain sequence. For example, in a case of the domain sequence illustrated in Fig. 7, w is $4 + 50 + 4 + 100 + 4 + 10 + 4 + 20 + 4 + 30 = 230$ (excluding the (A)n motif located at the most C-terminal side). Next, z/w (%) can be calculated by dividing z by w.

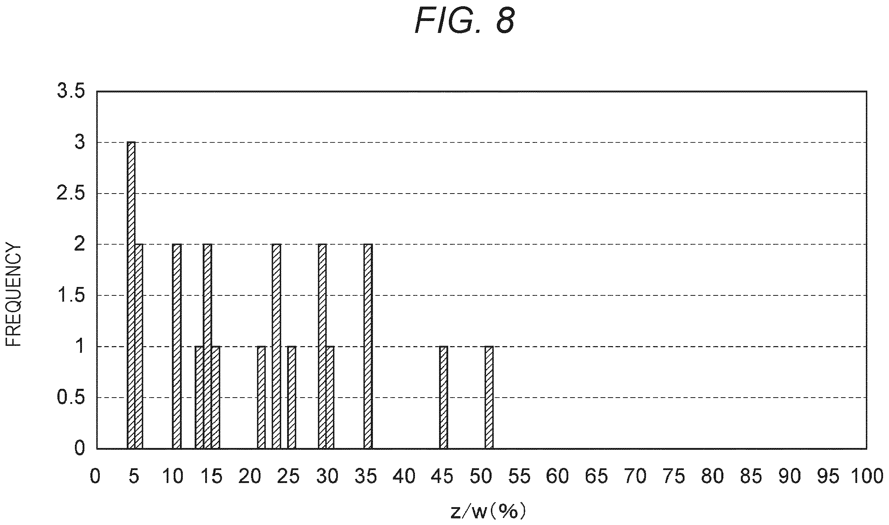

Here, z/w in naturally derived fibroin will be described. First, as described above, 663 types of fibroins (415 types of fibroins derived from spiders among them) were extracted by confirming fibroins with amino acid sequence information registered in NCBI GenBank by an exemplified method. The values of z/w were calculated by the calculation method described above, from amino acid sequences of naturally derived fibroins which contain a domain sequence represented by Formula 1: [(A)n motif-REP]m and in which the content ratio of the amino acid sequence consisting of GGX in the fibroin is 6% or less, among all the extracted fibroins. The results are illustrated in Fig. 8. In Fig. 8, the horizontal axis represents z/w (%), and the vertical axis represents a frequency. As is clear from Fig. 8, the values of z/w in naturally derived fibroin are all smaller than 50.9% (the largest value is 50.86%).

In the second modified fibroin, z/w is preferably 50.9% or more, more preferably 56.1% or more, still more preferably 58.7% or more, even still more preferably 70% or more, and even still more preferably 80% or more. The upper limit of z/w is not particularly limited, but may be 95% or less, for example.

The second modified fibroin can be obtained by, for example, substituting and modifying at least a part of a base sequence encoding a glycine residue from a cloned gene sequence of naturally derived fibroin so as to encode another amino acid residue. In this case, one glycine residue in a GGX motif or a GPGXX motif may be selected as the glycine residue to be modified, and substitution may be performed so that z/w is 50.9% or more. In addition, the second modified fibroin can also be obtained by, for example, designing an amino acid sequence satisfying each of the above aspects from the amino acid sequence of naturally derived fibroin, and chemically synthesizing a nucleic acid encoding the designed amino acid sequence. In any case, in addition to the modification corresponding to substitution of a glycine residue in REP with another amino acid residue from the amino acid sequence of naturally derived fibroin, modification of the amino acid sequence corresponding to substitution, deletion, insertion, and/or addition of one or a plurality of amino acid residues may be performed.

The above-described another amino acid residue is not particularly limited as long as it is an amino acid residue other than a glycine residue, but it is preferably a hydrophobic amino acid residue such as a valine (V) residue, a leucine (L) residue, an isoleucine (I) residue, a methionine (M) residue, a proline (P) residue, a phenylalanine (F) residue, or a tryptophan (W) residue, or a hydrophilic amino acid residue such as a glutamine (Q) residue, an asparagine (N) residue, a serine (S) residue, a lysine (K) residue, or a glutamic acid (E) residue, more preferably a valine (V) residue, a leucine (L) residue, an isoleucine (I) residue, a phenylalanine (F) residue, or a glutamine (Q) residue, and still more preferably a glutamine (Q) residue.

A more specific example of the second modified fibroin can include modified fibroin including (2-i) the amino acid sequence set forth in SEQ ID NO: 6 (Met-PRT380), SEQ ID NO: 7 (Met-PRT410), SEQ ID NO: 8 (Met-PRT525), or SEQ ID NO: 9 (Met-PRT799), or (2-ii) an amino acid sequence having 90% or more sequence identity with the amino acid sequence set forth in SEQ ID NO: 6, SEQ ID NO: 7, SEQ ID NO: 8, or SEQ ID NO: 9.

The modified fibroin of (2-i) will be described. The amino acid sequence set forth in SEQ ID NO: 6 is obtained by substituting all GGXs with GQX in REP of the amino acid sequence set forth in SEQ ID NO: 10 (Met-PRT313) corresponding to naturally derived fibroin. The amino acid sequence set forth in SEQ ID NO: 7 is obtained by deleting every other two (A)n motifs from the N-terminal side to the C-terminal side from the amino acid sequence set forth in SEQ ID NO: 6 and further inserting one [(A)n motif-REP] before the C-terminal sequence. The amino acid sequence set forth in SEQ ID NO: 8 is obtained by inserting two alanine residues at the C-terminal side of each (A)n motif of the amino acid sequence set forth in SEQ ID NO: 7 and further substituting a part of glutamine (Q) residues with a serine (S) residue to delete a part of amino acids at the C-terminal side so as to be almost the same as a molecular weight of SEQ ID NO: 7. The amino acid sequence set forth in SEQ ID NO: 9 is an amino acid sequence obtained by adding a predetermined hinge sequence and a His tag sequence to the C-terminus of a sequence obtained by repeating a region of 20 domain sequences (where several amino acid residues on the C-terminal side of the region are substituted) present in the amino acid sequence set forth in SEQ ID NO: 7 four times.

The value of z/w in the amino acid sequence set forth in SEQ ID NO: 10 (corresponding to naturally derived fibroin) is 46.8%. The values of z/w in the amino acid sequence set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are 58.7%, 70.1%, 66.1%, and 70.0%, respectively. In addition, the values of x/y in the amino acid sequences set forth in SEQ ID NO: 10, SEQ ID NO: 6, SEQ ID NO: 7, SEQ ID NO: 8, and SEQ ID NO: 9 at a Giza ratio (described below) of 1:1.8 to 11.3 are 15.0%, 15.0%, 93.4%, 92.7%, and 89.8%, respectively.

The modified fibroin of (2-i) may consist of the amino acid sequence set forth in SEQ ID NO: 6, SEQ ID NO: 7, SEQ ID NO: 8, or SEQ ID NO: 9.

The modified fibroin of (2-ii) includes an amino acid sequence having 90% or more sequence identity with the amino acid sequence set forth in SEQ ID NO: 6, SEQ ID NO: 7, SEQ ID NO: 8, or SEQ ID NO: 9. The modified fibroin of (2-ii) is also a protein containing a domain sequence represented by Formula 1: [(A)n motif-REP]m. The sequence identity is preferably 95% or more.

The modified fibroin of (2-ii) preferably has 90% or more sequence identity with the amino acid sequence set forth in SEQ ID NO: 6, SEQ ID NO: 7, SEQ ID NO: 8, or SEQ ID NO: 9, and z/w is preferably 50.9% or more in a case where the total number of amino acid residues in the amino acid sequence consisting of XGX (where X represents an amino acid residue other than glycine) in the REP is defined as z, and the total number of amino acid residues in the REP in the domain sequence is defined as w.

The second modified fibroin may have a tag sequence at either or both of the N-terminus and the C-terminus. This enables the modified fibroin to be isolated, immobilized, detected, and visualized.

The tag sequence may be, for example, an affinity tag utilizing specific affinity (binding property, affinity) with another molecule. A specific example of the affinity tag includes a histidine tag (His tag). The His tag is a short peptide in which about 4 to 10 histidine residues are arranged and has a property of specifically binding to a metal ion such as nickel. Thus, the His tag can be used for isolation of modified fibroin by chelating metal chromatography. A specific example of the tag sequence can include the amino acid sequence set forth in SEQ ID NO: 11 (amino acid sequence including a His tag sequence and a hinge sequence).

Also, a tag sequence such as glutathione-S-transferase (GST) that specifically binds to glutathione, and a maltose binding protein (MBP) that specifically binds to maltose can also be utilized.

Further, an "epitope tag" utilizing an antigen-antibody reaction can also be utilized. Adding a peptide (epitope) exhibiting antigenicity as a tag sequence allows an antibody against the epitope to be bound. Examples of the epitope tag include an HA (peptide sequence of hemagglutinin of influenza virus) tag, a myc tag, and a FLAG tag. The modified fibroin can easily be purified with high specificity by utilizing an epitope tag.

Moreover, it is possible to use a tag sequence which can be cleaved with a specific protease. The modified fibroin from which the tag sequence has been cleaved can be recovered by treating a protein adsorbed through the tag sequence with protease.

A more specific example of the modified fibroin including a tag sequence can include modified fibroin including (2-iii) the amino acid sequence set forth in SEQ ID NO: 12 (PRT380), SEQ ID NO: 13 (PRT410), SEQ ID NO: 14 (PRT525), or SEQ ID NO: 15 (PRT799), or (2-iv) an amino acid sequence having 90% or more sequence identity with the amino acid sequence set forth in SEQ ID NO: 12, SEQ ID NO: 13, SEQ ID NO: 14, or SEQ ID NO: 15.

Each of the amino acid sequences set forth in SEQ ID NO: 16 (PRT313), SEQ ID NO: 12, SEQ ID NO: 13, SEQ ID NO: 14, and SEQ ID NO: 15 is obtained by adding the amino acid sequence set forth in SEQ ID NO: 11 (including a His tag sequence and a hinge sequence) to the N-terminus of each of the amino acid sequences set forth in SEQ ID NO: 10, SEQ ID NO: 6, SEQ ID NO: 7, SEQ ID NO: 8, and SEQ ID NO: 9.

The modified fibroin of (2-iii) may consist of the amino acid sequence set forth in SEQ ID NO: 12, SEQ ID NO: 13, SEQ ID NO: 14, or SEQ ID NO: 15.

The modified fibroin of (2-iv) includes an amino acid sequence having 90% or more sequence identity with the amino acid sequence set forth in SEQ ID NO: 12, SEQ ID NO: 13, SEQ ID NO: 14, or SEQ ID NO: 15. The modified fibroin of (2-iv) is also a protein containing a domain sequence represented by Formula 1: [(A)n motif-REP]m. The sequence identity is preferably 95% or more.

The modified fibroin of (2-iv) preferably has 90% or more sequence identity with the amino acid sequence set forth in SEQ ID NO: 12, SEQ ID NO: 13, SEQ ID NO: 14, or SEQ ID NO: 15, and z/w is preferably 50.9% or more in a case where the total number of amino acid residues in the amino acid sequence consisting of XGX (where X represents an amino acid residue other than glycine) in the REP is defined as z, and the total number of amino acid residues in the REP in the domain sequence is defined as w.

The second modified fibroin may include a secretory signal for releasing the protein produced in the recombinant protein production system to the outside of a host. The sequence of the secretory signal can be appropriately set depending on the type of the host.

The domain sequence of the third modified fibroin has an amino acid sequence in which the content of the (A)n motif is reduced, as compared with naturally derived fibroin. It can be said that the domain sequence of the third modified fibroin has an amino acid sequence corresponding to an amino acid sequence in which at least one or a plurality of (A)n motifs are deleted, as compared with naturally derived fibroin.

The third modified fibroin may have an amino acid sequence corresponding to an amino acid sequence in which 10 to 40% of the (A)n motifs are deleted from naturally derived fibroin.

The domain sequence of the third modified fibroin may have an amino acid sequence corresponding to an amino acid sequence in which at least one (A)n motif of every one to three (A)n motifs is deleted from the N-terminal side to the C-terminal side, as compared with naturally derived fibroin.

The domain sequence of the third modified fibroin may have an amino acid sequence corresponding to an amino acid sequence in which deletion of at least two consecutive (A)n motifs and deletion of one (A)n motif are repeated in this order from the N-terminal side to the C-terminal side, as compared with naturally derived fibroin.

The third modified fibroin may have a domain sequence having an amino acid sequence corresponding to an amino acid sequence in which at least (A)n motif every other two positions is deleted from the N-terminal side to the C-terminal side.

The third modified fibroin contains a domain sequence represented by Formula 1: [(A)n motif-REP]m, and may have an amino acid sequence in which x/y is 20% or more, 30% or more, 40% or more, or 50% or more in a case where the numbers of amino acid residues in REPs of two adjacent [(A)n motif-REP] units are sequentially compared from the N-terminal side to the C-terminal side, and the number of amino acid residues in one REP having a smaller number of amino acid residues is defined as 1, the maximum value of the total value of the number of amino acid residues in the two adjacent [(A)n motif-REP] units where the ratio of the number of amino acid residues in the other REP is 1.8 to 11.3 is defined as x, and the total number of amino acid residues in the domain sequence is defined as y. The number of alanine residues with respect to the total number of amino acid residues in the (A)n motif is 83% or more, preferably 86% or more, more preferably 90% or more, still more preferably 95% or more, and even still more preferably 100% (meaning that the (A)n motif consists of only alanine residues).

The method of calculating x/y will be described in more detail with reference to Fig. 7. Fig. 7 illustrates a domain sequence excluding the N-terminal sequence and the C-terminal sequence from the modified fibroin. The domain

sequence has a sequence of (A)n motif-first REP (50 amino acid residues)-(A)n motif-second REP (100 amino acid residues)-(A)n motif-third REP (10 amino acid residues)-(A)n motif-fourth REP (20 amino acid residues)-(A)n motif-fifth REP (30 amino acid residues)-(A)n motif from the N-terminal side (left side).

The two adjacent [(A)n motif-REP] units are sequentially selected from the N-terminal side to the C-terminal side so as not to overlap. At this time, an unselected [(A)n motif-REP] unit may exist. Fig. 7 illustrates a pattern 1 (a comparison between the first REP and the second REP, and a comparison between the third REP and the fourth REP), a pattern 2 (a comparison between the first REP and the second REP, and a comparison between the fourth REP and the fifth REP), a pattern 3 (a comparison between the second REP and the third REP, and a comparison between the fourth REP and the fifth REP), and a pattern 4 (a comparison between the first REP and the second REP). There are other selection methods besides this.

Subsequently, the number of amino acid residues of each REP in the selected two adjacent [(A)n motif-REP] units is compared for each pattern. The comparison is performed by determining the ratio of the number of amino acid residues of the other REP in a case where one REP having a smaller number of amino acid residues is defined as 1. For example, in a case of comparing the first REP (50 amino acid residues) and the second REP (100 amino acid residues), the ratio of the number of amino acid residues of the second REP is 100/50 = 2 in a case where the first REP having a smaller number of amino acid residues is defined as 1. Similarly, in a case of comparing the fourth REP (20 amino acid residues) and the fifth REP (30 amino acid residues), the ratio of the number of amino acid residues of the fifth REP is 30/20 = 1.5 in a case where the fourth REP having a smaller number of amino acid residues is defined as 1.

In Fig. 7, a set of [(A)n motif-REP] units in which the ratio of the number of amino acid residues of the other REP is 1.8 to 11.3 in a case where one REP having a smaller number of amino acid residues is defined as 1 is indicated by a solid line. In the present specification, the ratio is referred to as a Giza ratio. A set of [(A)n motif-REP] units in which the ratio of the number of amino acid residues of the other REP is less than 1.8 or more than 11.3 in a case where one REP having a smaller number of amino acid residues is defined as 1 is indicated by a broken line.

In each pattern, the number of all amino acid residues of two adjacent [(A)n motif-REP] units indicated by solid lines (including not only the number of amino acid residues of REP but also the number of amino acid residues of the (A)n motif) is combined. Then, the total values combined are compared, and the total value of the pattern whose total value is the maximum (the maximum value of the total value) is defined as x. In the example illustrated in Fig. 7, the total value of the pattern 1 is the maximum.

Then, x/y (%) can be calculated by dividing x by the total number of amino acid residues y of the domain sequence.

In the third modified fibroin, x/y is preferably 50% or more, more preferably 60% or more, still more preferably 65% or more, even still more preferably 70% or more, even still more preferably 75% or more, and particularly preferably 80% or more. The upper limit of x/y is not particularly limited, but may be, for example, 100% or less. In a case where the Giza ratio is 1 : 1.9 to 11.3, x/y is preferably 89.6% or more; in a case where the Giza ratio is 1 : 1.8 to 3.4, x/y is preferably 77.1% or more; in a case where the Giza ratio is 1 : 1.9 to 8.4, x/y is preferably 75.9% or more; and in a case where the Giza ratio is 1 : 1.9 to 4.1, x/y is preferably 64.2% or more.

In a case where the third modified fibroin is modified fibroin in which at least seven of a plurality of (A)n motifs in the domain sequence consist of only alanine residues, x/y is preferably 46.4% or more, more preferably 50% or more, still more preferably 55% or more, even still more preferably 60% or more, even still more preferably 70% or more, and particularly preferably 80% or more. The upper limit of x/y is not particularly limited, but is only required to be 100% or less.

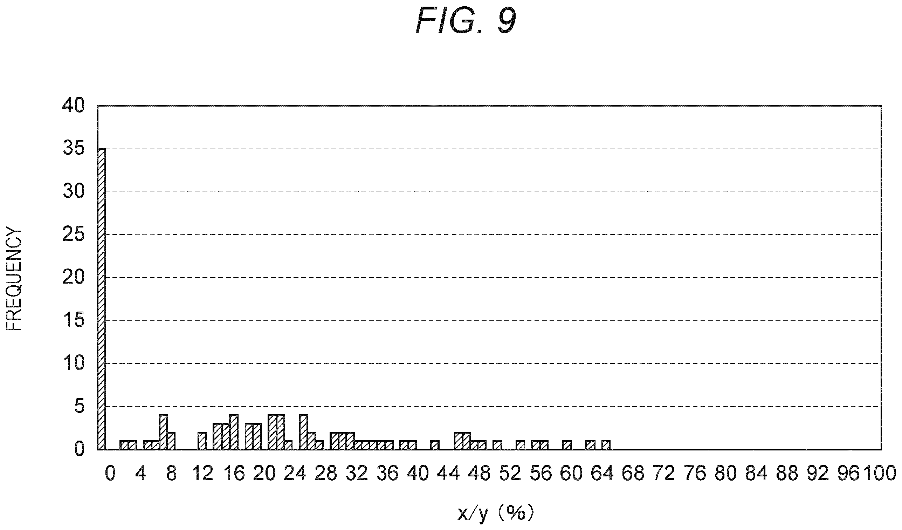

Here, x/y in naturally derived fibroin will be described. First, as described above, 663 types of fibroins (415 types of fibroins derived from spiders among them) were extracted by confirming fibroins with amino acid sequence information registered in NCBI GenBank by an exemplified method. The values of x/y were calculated by the calculation method described above, from amino acid sequences of naturally derived fibroins consisting of a domain sequence represented by Formula 1: [(A)n motif-REP]m, among all the extracted fibroins. The results in a case where the Giza ratio is 1:1.9 to 4.1 are illustrated in Fig. 9.

The horizontal axis in Fig. 9 represents x/y (%), and the vertical axis represents a frequency. As is clear from Fig. 9, the values of x/y in naturally derived fibroin are all smaller than 64.2% (the largest value is 64.14%).

The third modified fibroin can be obtained from, for example, a cloned gene sequence of naturally derived fibroin, by deleting one or a plurality of sequences encoding an (A)n motif so that x/y is 64.2% or more. In addition, for example, the third modified fibroin can also be obtained, from the amino acid sequence of naturally derived fibroin, by designing an amino acid sequence corresponding to an amino acid sequence in which one or a plurality of (A)n motifs are deleted so that x/y is 64.2% or more, and chemically synthesizing a nucleic acid encoding the designed amino acid sequence. In any case, in addition to the modification corresponding to deletion of the (A)n motif from the amino acid sequence of naturally derived fibroin, modification of the amino acid sequence corresponding to substitution, deletion, insertion, and/or addition of one or a plurality of amino acid residues may be performed.

A more specific example of the third modified fibroin can include modified fibroin including (3-i) the amino acid sequence set forth in SEQ ID NO: 17 (Met-PRT399), SEQ ID NO: 7 (Met-PRT410), SEQ ID NO: 8 (Met-PRT525), or SEQ

ID NO: 9 (Met-PRT799), or (3-ii) an amino acid sequence having 90% or more sequence identity with the amino acid sequence set forth in SEQ ID NO: 17, SEQ ID NO: 7, SEQ ID NO: 8, or SEQ ID NO: 9.

The modified fibroin of (3-i) will be described. The amino acid sequence set forth in SEQ ID NO: 17 is obtained by deleting every other two (A)n motifs from the N-terminal side to the C-terminal side from the amino acid sequence set forth in SEQ ID NO: 10 (Met-PRT313) corresponding to naturally derived fibroin and further inserting one [(A)n motif-REP] before the C-terminal sequence. The amino acid sequence set forth in SEQ ID NO: 7, SEQ ID NO: 8, or SEQ ID NO: 9 is as described in the second modified fibroin.

The value of x/y in the amino acid sequence set forth in SEQ ID NO: 10 (corresponding to naturally derived fibroin) at a Giza ratio of 1:1.8 to 11.3 is 15.0%. Both the value of x/y in the amino acid sequence set forth in SEQ ID NO: 17 and the value of x/y in the amino acid sequence set forth in SEQ ID NO: 7 are 93.4%. The value of x/y in the amino acid sequence set forth in SEQ ID NO: 8 is 92.7%. The value of x/y in the amino acid sequence set forth in SEQ ID NO: 9 is 89.8%. The values of z/w in the amino acid sequences set forth in SEQ ID NO: 10, SEQ ID NO: 17, SEQ ID NO: 7, SEQ ID NO: 8, and SEQ ID NO: 9 are 46.8%, 56.2%, 70.1%, 66.1%, and 70.0%, respectively.

The modified fibroin of (3-i) may consist of the amino acid sequence set forth in SEQ ID NO: 17, SEQ ID NO: 7, SEQ ID NO: 8, or SEQ ID NO: 9.

The modified fibroin of (3-ii) includes an amino acid sequence having 90% or more sequence identity with the amino acid sequence set forth in SEQ ID NO: 17, SEQ ID NO: 7, SEQ ID NO: 8, or SEQ ID NO: 9. The modified fibroin of (3-ii) is also a protein containing a domain sequence represented by Formula 1: [(A)n motif-REP]m. The sequence identity is preferably 95% or more.

It is preferable that the modified fibroin of (3-ii) has 90% or more sequence identity with the amino acid sequence set forth in SEQ ID NO: 17, SEQ ID NO: 7, SEQ ID NO: 8, or SEQ ID NO: 9, and x/y is 64.2% or more in a case where the numbers of amino acid residues in REPs of two adjacent [(A)n motif-REP] units are sequentially compared from the N-terminal side to the C-terminal side, and the number of amino acid residues in one REP having a small number of amino acid residues is defined as 1, the maximum value of the total value of the number of amino acid residues in the two adjacent [(A)n motif-REP] units where the ratio of the number of amino acid residues in the other REP is 1.8 to 11.3 (the Giza ratio is 1:1.8 to 11.3) is defined as x, and the total number of amino acid residues in the domain sequence is defined as y.

The third modified fibroin may include the above-described tag sequence at either or both of the N-terminus and the C-terminus.

A more specific example of the modified fibroin including a tag sequence can include modified fibroin including (3-iii) the amino acid sequence set forth in SEQ ID NO: 18 (PRT399), SEQ ID NO: 13 (PRT410), SEQ ID NO: 14 (PRT525), or SEQ ID NO: 15 (PRT799), or (3-iv) an amino acid sequence having 90% or more sequence identity with the amino acid sequence set forth in SEQ ID NO: 18, SEQ ID NO: 13, SEQ ID NO: 14, or SEQ ID NO: 15.

Each of the amino acid sequences set forth in SEQ ID NO: 18, SEQ ID NO: 13, SEQ ID NO: 14, and SEQ ID NO: 15 is obtained by adding the amino acid sequence set forth in SEQ ID NO: 11 (including a His tag sequence and a hinge sequence) to the N-terminus of each of the amino acid sequences set forth in SEQ ID NO: 17, SEQ ID NO: 7, SEQ ID NO: 8, and SEQ ID NO: 9.

The modified fibroin of (3-iii) may consist of the amino acid sequence set forth in SEQ ID NO: 18, SEQ ID NO: 13, SEQ ID NO: 14, or SEQ ID NO: 15.

The modified fibroin of (3-iv) includes an amino acid sequence having 90% or more sequence identity with the amino acid sequence set forth in SEQ ID NO: 18, SEQ ID NO: 13, SEQ ID NO: 14, or SEQ ID NO: 15. The modified fibroin of (3-iv) is also a protein containing a domain sequence represented by Formula 1: [(A)n motif-REP]m. The sequence identity is preferably 95% or more.

It is preferable that the modified fibroin of (3-iv) has 90% or more sequence identity with the amino acid sequence set forth in SEQ ID NO: 18, SEQ ID NO: 13, SEQ ID NO: 14, or SEQ ID NO: 15, and x/y is 64.2% or more in a case where the number of amino acid residues in REPs in two adjacent [(A)n motif-REP] units are sequentially compared from the N-terminal side to the C-terminal side, and the number of amino acid residues in one REP having a small number of amino acid residues is defined as 1, the maximum value of the total value of the number of amino acid residues in the two adjacent [(A)n motif-REP] units where the ratio of the number of amino acid residues in the other REP is 1.8 to 11.3 is defined as x, and the total number of amino acid residues in the domain sequence is defined as y.

The third modified fibroin may include a secretory signal for releasing the protein produced in the recombinant protein production system to the outside of a host. The sequence of the secretory signal can be appropriately set depending on the type of the host.

The domain sequence of the fourth modified fibroin has an amino acid sequence in which the content of an (A)n motif and the content of glycine residues are reduced, as compared with naturally derived fibroin. It can be said that the domain sequence of the fourth modified fibroin has an amino acid sequence corresponding to an amino acid sequence in which at least one or a plurality of (A)n motifs are deleted and at least one or a plurality of glycine residues in REP are substituted with another amino acid residue, as compared with naturally derived fibroin. That is, the fourth modified fibroin is modified fibroin having the characteristics of the above-described second modified fibroin and third modified fibroin.

Specific aspects thereof and the like are as in the descriptions for the second modified fibroin and the third modified fibroin.

A more specific example of the fourth modified fibroin can include modified fibroin including (4-i) the amino acid sequence set forth in SEQ ID NO: 7 (Met-PRT410), SEQ ID NO: 8 (Met-PRT525), SEQ ID NO: 9 (Met-PRT799), SEQ ID NO: 13 (PRT410), SEQ ID NO: 14 (PRT525), or SEQ ID NO: 15 (PRT799), or (4-ii) an amino acid sequence having 90% or more sequence identity with the amino acid sequence set forth in SEQ ID NO: 7, SEQ ID NO: 8, SEQ ID NO: 9, SEQ ID NO: 13, SEQ ID NO: 14, or SEQ ID NO: 15. Specific aspects of the modified fibroin including the amino acid sequence set forth in SEQ ID NO: 7, SEQ ID NO: 8, SEQ ID NO: 9, SEQ ID NO: 13, SEQ ID NO: 14, or SEQ ID NO: 15 are as described above.

The domain sequence of the fifth modified fibroin may have an amino acid sequence including a region locally having a high hydropathy index corresponding to an amino acid sequence in which one or a plurality of amino acid residues in REP are substituted with amino acid residues having a high hydropathy index and/or one or a plurality of amino acid residues having a high hydropathy index are inserted into REP, as compared with naturally derived fibroin.

The region locally having a high hydropathy index preferably consists of consecutive two to four amino acid residues.

The above-described amino acid residue having a high hydropathy index is more preferably an amino acid residue selected from isoleucine (I), valine (V), leucine (L), phenylalanine (F), cysteine (C), methionine (M), and alanine (A).

The fifth modified fibroin may be further subjected to modification of an amino acid sequence corresponding to substitution, deletion, insertion, and/or addition of one or a plurality of amino acid residues as compared with naturally derived fibroin, in addition to modification corresponding to substitution of one or a plurality of amino acid residues in REP with amino acid residues having a high hydropathy index and/or insertion of one or a plurality of amino acid residues having a high hydropathy index into REP, as compared with naturally derived fibroin.

The fifth modified fibroin can be obtained by, for example, substituting one or a plurality of hydrophilic amino acid residues in REP (for example, amino acid residues having a negative hydropathy index) with hydrophobic amino acid residues (for example, amino acid residues having a positive hydropathy index) from a cloned gene sequence of naturally derived fibroin, and/or inserting one or a plurality of hydrophobic amino acid residues into REP. In addition, the fifth modified fibroin can be obtained by, for example, designing an amino acid sequence corresponding to an amino acid sequence in which one or a plurality of hydrophilic amino acid residues in REP are substituted with hydrophobic amino acid residues from an amino acid sequence of naturally derived fibroin, and/or one or a plurality of hydrophobic amino acid residues are inserted into REP, and chemically synthesizing a nucleic acid encoding the designed amino acid sequence. In any case, in addition to modification corresponding to substitution of one or a plurality of hydrophilic amino acid residues in REP with hydrophobic amino acid residues from an amino acid sequence of naturally derived fibroin, and/or insertion of one or a plurality of hydrophobic amino acid residues into REP, modification of an amino acid sequence corresponding to substitution, deletion, insertion, and/or addition of one or a plurality of amino acid residues may be further performed.

The fifth modified fibroin contains a domain sequence represented by Formula 1: [(A)n motif-REP]m, and may have an amino acid sequence in which p/q is 6.2% or more in a case where in all REPs included in a sequence excluding the sequence from the (A)n motif located at the most C-terminal side to the C-terminus of the domain sequence from the domain sequence, the total number of amino acid residues included in a region where the average value of hydropathy indices of four consecutive amino acid residues is 2.6 or more is defined as p, and the total number of amino acid residues included in the sequence excluding the sequence from the (A)n motif located at the most C-terminal side to the C-terminus of the domain sequence from the domain sequence is defined as q.

} For the hydropathy index of the amino acid residue, a publicly known index (Hydropathy index: Kyte J, & Doolittle R (1982) "A simple method for displaying the hydropathic character of a protein", J. Mol. Biol., 157, pp.105-132) is used. Specifically, the hydropathy index (hereinafter, also referred to as "HI") of each amino acid is as shown in Table 1.

[Table 1]

| Amino acid | HI | Amino acid | HI |
|---|---|---|---|
| Isoleucine (Ile) | 4.5 | Tryptophane (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |

(continued)

| Amino acid | HI | Amino acid | HI |
|---|---|---|---|
| Threonine (Thr) | -0.7 | Lysine (Lys) | -3.9 |
| Serine (Ser) | -0.8 | Arginine (Arg) | -4.5 |

The method of calculating p/q will be described in more detail. In the calculation, a sequence excluding the sequence from the (A)n motif located at the most C-terminal side to the C-terminus of the domain sequence from the domain sequence represented by Formula 1 [(A)n motif-REP]m (hereinafter also referred to as "sequence A") is used. First, in all REPs included in the sequence A, the average values of hydropathy indices of four consecutive amino acid residues are calculated. The average value of the hydropathy indices is determined by dividing the total sum of HIs of respective amino acid residues included in the four consecutive amino acid residues by 4 (number of amino acid residues). The average value of the hydropathy indices is determined for all of the four consecutive amino acid residues (each of the amino acid residues is used for calculating the average value 1 to 4 times). Then, a region where the average value of the hydropathy indices of the four consecutive amino acid residues is 2.6 or more is specified. Even in a case where a certain amino acid residue corresponds to the "four consecutive amino acid residues having an average value of hydropathy indices of 2.6 or more" multiple times, the amino acid residue is included as one amino acid residue in the region. The total number of amino acid residues included in the region is p. Also, the total number of amino acid residues included in the sequence A is q.

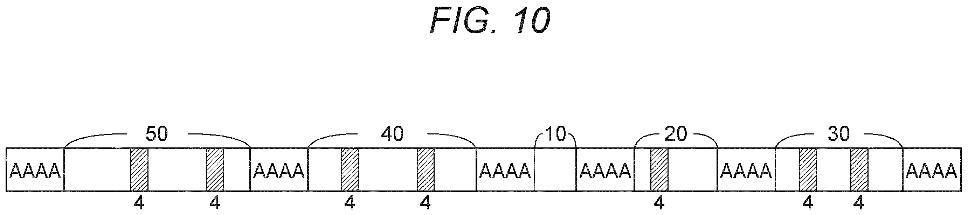

For example, in a case where the "four consecutive amino acid residues having an average value of hydropathy indices of 2.6 or more" are extracted from 20 places (no overlap), in the region where the average value of hydropathy indices of four consecutive amino acid residues is 2.6 or more, 20 of the four consecutive amino acid residues (no overlap) are included, and thus p is 20 × 4 = 80. In addition, for example, in a case where two of the "four consecutive amino acid residues having an average value of the hydropathy indices of 2.6 or more" overlap by only one amino acid residue, in the region where the average value of the hydropathy indices of four consecutive amino acid residues is 2.6 or more, the number of amino acid residues is 7 (p = 2 × 4 - 1 = 7, "-1" is the deduction of overlap). For example, in a case of the domain sequence illustrated in Fig. 10, seven sets of "four consecutive amino acid residues having an average value of hydropathy indices of 2.6 or more" are present without overlaps, and thus, p is 7 × 4 = 28. Furthermore, for example, in the case of the domain sequence illustrated in Fig. 10, q is 4 + 50 + 4 + 40 + 4 + 10 + 4 + 20 + 4 + 30 = 170 (the (A)n motif located at the end of the C-terminal side is excluded). Next, p/q (%) can be calculated by dividing p by q. In the case of Fig. 10, p/q is 28/170 = 16.47%.

In the fifth modified fibroin, p/q is preferably 6.2% or more, more preferably 7% or more, still more preferably 10% or more, even still more preferably 20% or more, and even still more preferably 30% or more. The upper limit of p/q is not particularly limited, but may be 45% or less, for example.

The fifth modified fibroin can be obtained by, for example, substituting one or a plurality of hydrophilic amino acid residues in REP (for example, amino acid residues having a negative hydropathy index) with hydrophobic amino acid residues (for example, amino acid residues having a positive hydropathy index) so that a cloned amino acid sequence of naturally derived fibroin satisfies the condition of p/q, and/or modifying the cloned amino acid sequence of naturally derived fibroin into an amino acid sequence including a region locally having a high hydropathy index by inserting one or a plurality of hydrophobic amino acid residues into REP. In addition, the fifth modified fibroin can also be obtained by, for example, designing an amino acid sequence satisfying the condition of p/q from the amino acid sequence of naturally derived fibroin, and chemically synthesizing a nucleic acid encoding the designed amino acid sequence. In any case, modification corresponding to substitution, deletion, insertion, and/or addition of one or a plurality of amino acid residues may also be performed, in addition to modification corresponding to substitution of one or a plurality of amino acid residues in REP with amino acid residues having a high hydropathy index, and/or insertion of one or a plurality of amino acid residues having a high hydropathy index into REP, as compared with naturally derived fibroin.

The amino acid residue having a high hydropathy index is not particularly limited, but is preferably isoleucine (I), valine (V), leucine (L), phenylalanine (F), cysteine (C), methionine (M), and alanine (A), and more preferably valine (V), leucine (L), and isoleucine (I).

A more specific example of the fifth modified fibroin can include modified fibroin including (5-i) the amino acid sequence set forth in SEQ ID NO: 19 (Met-PRT720), SEQ ID NO: 20 (Met-PRT665), or SEQ ID NO: 21 (Met-PRT666), or (5-ii) an amino acid sequence having 90% or more sequence identity with the amino acid sequence set forth in SEQ ID NO: 19, SEQ ID NO: 20, or SEQ ID NO: 21.

The modified fibroin of (5-i) will be described. The amino acid sequence set forth in SEQ ID NO: 19 is obtained by inserting an amino acid sequence consisting of three amino acid residues (VLI) at two sites for each REP into the amino acid sequence set forth in SEQ ID NO: 7 (Met-PRT410), except for the domain sequence at the end on the C-terminal side, and further substituting a part of glutamine (Q) residues with serine (S) residues, and deleting a part of amino acids on the

C-terminal side. The amino acid sequence set forth in SEQ ID NO: 20 is obtained by inserting the amino acid sequence consisting of three amino acid residues (VLI) at one site for each REP into the amino acid sequence set forth in SEQ ID NO: 8 (Met-PRT525). The amino acid sequence set forth in SEQ ID NO: 21 is obtained by inserting the amino acid sequence consisting of three amino acid residues (VLI) at two sites for each REP into the amino acid sequence set forth in SEQ ID NO: 8.

The modified fibroin of (5-i) may consist of the amino acid sequence set forth in SEQ ID NO: 19, SEQ ID NO: 20, or SEQ ID NO: 21.

The modified fibroin of (5-ii) includes an amino acid sequence having 90% or more sequence identity with the amino acid sequence set forth in SEQ ID NO: 19, SEQ ID NO: 20, or SEQ ID NO: 21. The modified fibroin of (5-ii) is also a protein containing a domain sequence represented by Formula 1: [(A)n motif-REP]m. The sequence identity is preferably 95% or more.

It is preferable that the modified fibroin of (5-ii) has 90% or more sequence identity with the amino acid sequence set forth in SEQ ID NO: 19, SEQ ID NO: 20, or SEQ ID NO: 21, and p/q is 6.2% or more in a case where in all REPs included in a sequence excluding the sequence from the (A)n motif located at the most C-terminal side to the C-terminus of the domain sequence from the domain sequence, the total number of amino acid residues included in a region where the average value of hydropathy indices of four consecutive amino acid residues is 2.6 or more is defined as p, and the total number of amino acid residues included in the sequence excluding the sequence from the (A)n motif located at the most the C-terminal side to the C-terminus of the domain sequence from the domain sequence is defined as q.

The fifth modified fibroin may include a tag sequence at either or both of the N-terminus and the C-terminus.

A more specific example of the modified fibroin including a tag sequence can include modified fibroin including (5-iii) the amino acid sequence set forth in SEQ ID NO: 22 (PRT720), SEQ ID NO: 23 (PRT665), or SEQ ID NO: 24 (PRT666), or (5-iv) an amino acid sequence having 90% or more sequence identity with the amino acid sequence set forth in SEQ ID NO: 22, SEQ ID NO: 23, or SEQ ID NO: 24.

Each of the amino acid sequences set forth in SEQ ID NO: 22, SEQ ID NO: 23, and SEQ ID NO: 24 is obtained by adding the amino acid sequence set forth in SEQ ID NO: 11 (including a His tag sequence and a hinge sequence) to the N-terminus of each of the amino acid sequences set forth in SEQ ID NO: 19, SEQ ID NO: 20, and SEQ ID NO: 21.

The modified fibroin of (5-iii) may consist of the amino acid sequence set forth in SEQ ID NO: 22, SEQ ID NO: 23, or SEQ ID NO: 24.

The modified fibroin of (5-iv) includes an amino acid sequence having 90% or more sequence identity with the amino acid sequence set forth in SEQ ID NO: 22, SEQ ID NO: 23, or SEQ ID NO: 24. The modified fibroin of (5-iv) is also a protein containing a domain sequence represented by Formula 1: [(A)n motif-REP]m. The sequence identity is preferably 95% or more.

It is preferable that the modified fibroin of (5-iv) has 90% or more sequence identity with the amino acid sequence set forth in SEQ ID NO: 22, SEQ ID NO: 23, or SEQ ID NO: 24, and p/q is 6.2% or more in a case where in all REPs included in a sequence excluding the sequence from the (A)n motif located at the most C-terminal side to the C-terminus of the domain sequence from the domain sequence, the total number of amino acid residues included in a region where the average value of hydropathy indices of four consecutive amino acid residues is 2.6 or more is defined as p, and the total number of amino acid residues included in the sequence excluding the sequence from the (A)n motif located at the most C-terminal side to the C-terminus of the domain sequence from the domain sequence is defined as q.

The fifth modified fibroin may include a secretory signal for releasing the protein produced in the recombinant protein production system to the outside of a host. The sequence of the secretory signal can be appropriately set depending on the type of the host.

The sixth modified fibroin has an amino acid sequence in which the content of glutamine residues is reduced, as compared with naturally derived fibroin.

In the sixth modified fibroin, at least one motif selected from a GGX motif and a GPGXX motif is preferably included in the amino acid sequence of REP.

In a case where the sixth modified fibroin has the GPGXX motif in REP, a content rate of the GPGXX motif is usually 1% or more, may also be 5% or more, and preferably 10% or more. The upper limit of the content rate of the GPGXX motif is not particularly limited, and may be 50% or less, or may also be 30% or less.

In the present specification, the "content rate of the GPGXX motif" is a value calculated by the following method. The content rate of the GPGXX motif in fibroin (modified fibroin or naturally derived fibroin) containing a domain sequence represented by Formula 1: [(A)n motif-REP]m or Formula 2: [(A)n motif-REP]m-(A)n motif is calculated as s/t, in a case where the number obtained by tripling the total number of GPGXX motifs in regions of all REPs included in a sequence excluding the sequence from the (A)n motif located at the most C-terminal side to the C-terminus of the domain sequence from the domain sequence (that is, corresponding to the total number of G and P in the GPGXX motifs) is defined as s, and the total number of amino acid residues in all REPs excluding a sequence from the (A)n motif located at the most C-terminal side to the C-terminus of the domain sequence from the domain sequence and further excluding the (A)n motifs is defined as t.

In the calculation of the content rate of the GPGXX motif, the "sequence excluding the sequence from the (A)n motif located at the most C-terminal side to the C-terminus of the domain sequence from the domain sequence" is used to exclude the effect occurring due to the fact that the "sequence from the (A)n motif located at the most C-terminal side to the C-terminus of the domain sequence" (a sequence corresponding to REP) may include a sequence having a low correlation with the sequence characteristic of fibroin, which influences the calculation result of the content rate of the GPGXX motif in a case where m is small (that is, in a case where the domain sequence is short). Incidentally, in a case where the "GPGXX motif" is located at the C-terminus of REP, even when "XX" is "AA", for example, it is treated as the "GPGXX motif".

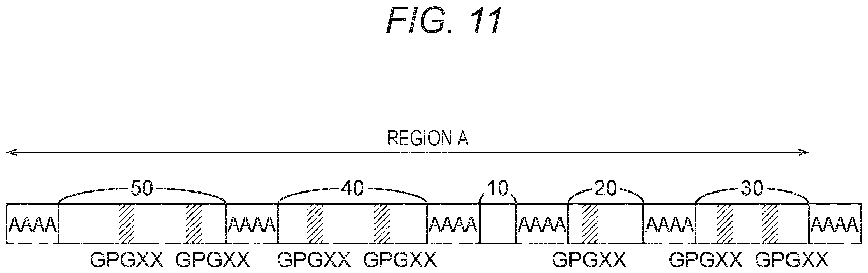

Fig. 11 is a schematic diagram illustrating a domain sequence of modified fibroin. The method for calculating the content rate of the GPGXX motif will be specifically described with reference to Fig. 11. First, in the domain sequence of the modified fibroin illustrated in Fig. 11 (which is the "[(A)n motif-REP]m-(A)n motif" type), all REPs are included in the "sequence excluding the sequence from the (A)n motif located at the most C-terminal side to the C-terminus of the domain sequence from the domain sequence" (in Fig. 11, the sequence indicated as a "region A"), and therefore, the number of the GPGXX motifs for calculating s is 7, and s is $7 \times 3 = 21$. Similarly, since all REPs are included in the "sequence excluding the sequence from the (A)n motif located at the most C-terminal side to the C-terminus of the domain sequence from the domain sequence" (in Fig. 11, the sequence indicated as the "region A"), the total number t of the amino acid residues in all REPs when the (A)n motifs are further excluded from the sequence is 50 + 40 + 10 + 20 + 30 = 150. Next, s/t (%) can be calculated by dividing s by t, and in the case of the modified fibroin of Fig. 11, s/t is 21/150 = 14.0%.

In the sixth modified fibroin, a content rate of the glutamine residue is preferably 9% or less, more preferably 7% or less, still more preferably 4% or less, and particularly preferably 0%.

In the present specification, the "content rate of the glutamine residue" is a value calculated by the following method. The content rate of the glutamine residue in fibroin (modified fibroin or naturally derived fibroin) containing a domain sequence represented by Formula 1: [(A)n motif-REP]m or Formula 2: [(A)n motif-REP]m-(A)n motif is calculated as u/t, in a case where the total number of glutamine residues included in regions of all REPs included in a sequence excluding the sequence from the (A)n motif located at the most C-terminal side to the C-terminus of the domain sequence from the domain sequence (a sequence corresponding to the "region A" in Fig. 11) is defined as u, and the total number of amino acid residues in all REPs in the sequence excluding the sequence from the (A)n motif located at the most C-terminal side to the C-terminus of the domain sequence from the domain sequence and further excluding the (A)n motifs is defined as t. In the calculation of the content rate of the glutamine residue, the reason for targeting the "sequence excluding the sequence from the (A)n motif located at the most C-terminal side to the C-terminus of the domain sequence from the domain sequence" is the same as the reason described above.

The domain sequence of the sixth modified fibroin may have an amino acid sequence corresponding to an amino acid sequence in which one or a plurality of glutamine residues in REP are deleted, or one or a plurality of glutamine residues are substituted with another amino acid residue, as compared with naturally derived fibroin.

The "another amino acid residue" may be an amino acid residue other than the glutamine residue, but is preferably an amino acid residue having a higher hydropathy index than that of the glutamine residue. The hydropathy index of the amino acid residue is as shown in Table 1.

As shown in Table 1, examples of the amino acid residue having a higher hydropathy index than that of the glutamine residue include amino acid residues selected from isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H). Among them, the amino acid residue is more preferably an amino acid residue selected from isoleucine (I), valine (V), leucine (L), phenylalanine (F), cysteine (C), methionine (M), and alanine (A), and still more preferably an amino acid residue selected from isoleucine (I), valine (V), leucine (L), and phenylalanine (F).

In the sixth modified fibroin, the hydrophobicity of REP is preferably -0.8 or more, more preferably -0.7 or more, still more preferably 0 or more, even still more preferably 0.3 or more, and particularly preferably 0.4 or more. The upper limit of the hydrophobicity of REP is not particularly limited, but may be 1.0 or less or 0.7 or less.

In the present specification, the "hydrophobicity of REP" is a value calculated by the following method. The hydrophobicity of REP in fibroin containing a domain sequence represented by Formula 1: [(A)n motif-REP]m or Formula 2: [(A)n motif-REP]m-(A)n motif (modified fibroin or naturally derived fibroin) is calculated as v/t, in a case where the sum of hydropathy indices of amino acid residues in regions of all REPs included in a sequence excluding the sequence from the (A)n motif located at the most C-terminal side to the C-terminus of the domain sequence from the domain sequence (a sequence corresponding to the "region A" in Fig. 11) is defined as v, and the total number of amino acid residues in all REPs in the sequence excluding the sequence from the (A)n motif located at the most C-terminal side to the C-terminus of the domain sequence from the domain sequence and further excluding the (A)n motifs is defined as t. In the calculation of the hydrophobicity of REP, the reason for targeting the "sequence excluding the sequence from the (A)n motif located at the most C-terminal side to the C-terminus of the domain sequence from the domain sequence" is the same as the reason described above.

The domain sequence of the sixth modified fibroin may be further subjected to modification of an amino acid sequence corresponding to substitution, deletion, insertion, and/or addition of one or a plurality of amino acid residues, in

addition to modification corresponding to deletion of one or a plurality of glutamine residues in REP, and/or substitution of one or a plurality of glutamine residues in REP with another amino acid residue, as compared with naturally derived fibroin.

The sixth modified fibroin can be obtained by, for example, deleting one or a plurality of glutamine residues in REP from a cloned gene sequence of naturally derived fibroin, and/or substituting one or a plurality of glutamine residues in REP with another amino acid residue. In addition, the sixth modified fibroin can be obtained by, for example, designing an amino acid sequence corresponding to an amino acid sequence in which one or a plurality of glutamine residues in REP are deleted from an amino acid sequence of naturally derived fibroin, and/or one or a plurality of glutamine residues in REP are substituted with another amino acid residue, and chemically synthesizing a nucleic acid encoding the designed amino acid sequence.

A more specific example of the sixth modified fibroin can include modified fibroin including (6-i) the amino acid sequence set forth in SEQ ID NO: 25 (Met-PRT888), SEQ ID NO: 26 (Met-PRT965), SEQ ID NO: 27 (Met-PRT889), SEQ ID NO: 28 (Met-PRT916), SEQ ID NO: 29 (Met-PRT918), SEQ ID NO: 30 (Met-PRT699), SEQ ID NO: 31 (Met-PRT698), SEQ ID NO: 32 (Met-PRT966), SEQ ID NO: 41 (Met-PRT917), or SEQ ID NO: 42 (Met-PRT1028), and modified fibroin including (6-ii) an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin of (6-i) will be described. The amino acid sequence set forth in SEQ ID NO: 25 is obtained by substituting all QQs in the amino acid sequence set forth in SEQ ID NO: 7 (Met-PRT410) with VL. The amino acid sequence set forth in SEQ ID NO: 26 is obtained by substituting all QQs in the amino acid sequence set forth in SEQ ID NO: 7 with TS and substituting the remaining Q with A. The amino acid sequence set forth in SEQ ID NO: 27 is obtained by substituting all QQs in the amino acid sequence set forth in SEQ ID NO: 7 with VL and substituting the remaining Q with I. The amino acid sequence set forth in SEQ ID NO: 28 is obtained by substituting all QQs in the amino acid sequence set forth in SEQ ID NO: 7 with VI and substituting the remaining Q with L. The amino acid sequence set forth in SEQ ID NO: 29 is obtained by substituting all QQs in the amino acid sequence set forth in SEQ ID NO: 7 with VF and substituting the remaining Q with I.

The amino acid sequence set forth in SEQ ID NO: 30 is obtained by substituting all QQs in the amino acid sequence set forth in SEQ ID NO: 8 (Met-PRT525) with VL. The amino acid sequence set forth in SEQ ID NO: 31 is obtained by substituting all QQs in the amino acid sequence set forth in SEQ ID NO: 8 with VL and substituting the remaining Q with I.

The amino acid sequence set forth in SEQ ID NO: 32 is obtained by substituting, with VF, all QQs in a sequence obtained by repeating a region of 20 domain sequences present in the amino acid sequence set forth in SEQ ID NO: 7 (Met-PRT410) two times and substituting the remaining Q with I.

The amino acid sequence set forth in SEQ ID NO: 41 (Met-PRT917) is obtained by substituting all QQs in the amino acid sequence set forth in SEQ ID NO: 7 with LI and substituting the remaining Q with V. The amino acid sequence set forth in SEQ ID NO: 42 (Met-PRT1028) is obtained by substituting all QQs in the amino acid sequence set forth in SEQ ID NO: 7 with IF and substituting the remaining Q with T.

The content rate of the glutamine residue in each of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42 is 9% or less (Table 2).

[Table 2]

| Modified fibroin | Content rate of glutamine residue | Content rate of GPGXX motif | Hydrophobicity of REP |
|---|---|---|---|
| Met-PRT410 (SEQ ID NO: 7) | 17.7% | 27.9% | -1.52 |
| Met-PRT888 (SEQ ID NO: 25) | 6.3% | 27.9% | -0.07 |
| Met-PRT965 (SEQ ID NO: 26) | 0.0% | 27.9% | -0.65 |
| Met-PRT889 (SEQ ID NO: 27) | 0.0% | 27.9% | 0.35 |
| Met-PRT916 (SEQ ID NO: 28) | 0.0% | 27.9% | 0.47 |
| Met-PRT918 (SEQ ID NO: 29) | 0.0% | 27.9% | 0.45 |
| Met-PRT699 (SEQ ID NO: 30) | 3.6% | 26.4% | -0.78 |
| Met-PRT698 (SEQ ID NO: 31) | 0.0% | 26.4% | -0.03 |
| Met-PRT966 (SEQ ID NO: 32) | 0.0% | 28.0% | 0.35 |
| Met-PRT917 (SEQ ID NO: 41) | 0.0% | 27.9% | 0.46 |

(continued)

| Modified fibroin | Content rate of glutamine residue | Content rate of GPGXX motif | Hydrophobicity of REP |
|---|---|---|---|
| Met-PRT1028 (SEQ ID NO: 42) | 0.0% | 28.1% | 0.05 |

The modified fibroin of (6-i) may consist of the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin of (6-ii) includes an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42. The modified fibroin of (6-ii) is also a protein containing a domain sequence represented by Formula 1: [(A)n motif-REP]m or Formula 2: [(A)n motif-REP]m-(A)n motif. The sequence identity is preferably 95% or more.

In the modified fibroin of (6-ii), the content rate of the glutamine residue is preferably 9% or less. In the modified fibroin of (6-ii), the content rate of the GPGXX motif is preferably 10% or more.

The sixth modified fibroin may have a tag sequence at either or both of the N-terminus and the C-terminus. This enables the modified fibroin to be isolated, immobilized, detected, and visualized.

A more specific example of the modified fibroin including a tag sequence can include modified fibroin including (6-iii) the amino acid sequence set forth in SEQ ID NO: 33 (PRT888), SEQ ID NO: 34 (PRT965), SEQ ID NO: 35 (PRT889), SEQ ID NO: 36 (PRT916), SEQ ID NO: 37 (PRT918), SEQ ID NO: 38 (PRT699), SEQ ID NO: 39 (PRT698), SEQ ID NO: 40 (PRT966), SEQ ID NO: 43 (PRT917), or SEQ ID NO: 44 (PRT1028), or modified fibroin including (6-iv) an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

Each of the amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 is obtained by adding the amino acid sequence set forth in SEQ ID NO: 11 (including a His tag sequence and a hinge sequence) to the N-terminus of each of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42. Since only the tag sequence is added to the N-terminus, the content rate of the glutamine residue is not changed, and the content rate of the glutamine residue in each of the amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 is 9% or less (Table 3).

[Table 3]

| Modified fibroin | Content rate of glutamine residue | Content rate of GPGXX motif | Hydrophobicity of REP |
|---|---|---|---|
| PRT888 (SEQ ID NO: 33) | 6.3% | 27.9% | -0.07 |
| PRT965 (SEQ ID NO: 34) | 0.0% | 27.9% | -0.65 |
| PRT889 (SEQ ID NO: 35) | 0.0% | 27.9% | 0.35 |
| PRT916 (SEQ ID NO: 36) | 0.0% | 27.9% | 0.47 |
| PRT918 (SEQ ID NO: 37) | 0.0% | 27.9% | 0.45 |
| PRT699 (SEQ ID NO: 38) | 3.6% | 26.4% | -0.78 |
| PRT698 (SEQ ID NO: 39) | 0.0% | 26.4% | -0.03 |
| PRT966 (SEQ ID NO: 40) | 0.0% | 28.0% | 0.35 |
| PRT917 (SEQ ID NO: 43) | 0.0% | 27.9% | 0.46 |
| PRT1028 (SEQ ID NO: 44) | 0.0% | 28.1% | 0.05 |

The modified fibroin of (6-iii) may consist of the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

The modified fibroin of (6-iv) includes an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44. The modified fibroin of (6-iv) is also a protein containing a domain sequence represented by Formula 1: [(A)n motif-REP]m or Formula 2: [(A)n motif-REP]m- (A)n motif. The sequence identity is preferably 95% or more.
In the modified fibroin of (6-iv), the content rate of the glutamine residue is preferably 9% or less. In the modified fibroin of (6-iv), the content rate of the GPGXX motif is preferably 10% or more.
The sixth modified fibroin may include a secretory signal for releasing the protein produced in the recombinant protein production system to the outside of a host. The sequence of the secretory signal can be appropriately set depending on the type of the host.
The modified fibroin may also be modified fibroin having at least two or more characteristics among the characteristics of the first modified fibroin, the second modified fibroin, the third modified fibroin, the fourth modified fibroin, the fifth modified fibroin, and the sixth modified fibroin.
The modified fibroin according to the present embodiment can be produced by an ordinary method using a nucleic acid encoding the modified fibroin. The nucleic acid encoding the modified fibroin may be chemically synthesized based on nucleotide sequence information, or may be synthesized using a PCR method or the like. Isolation and purification of the produced modified fibroin can be performed by a commonly used method.

[Method for producing protein fiber fabric]

The method for producing a protein fiber fabric according to the present embodiment includes a step of preparing a fabric using the protein fibers obtained by the method for producing a protein fiber according to the present invention. A method for preparing a fabric using protein fibers is not particularly limited, and a known method can be used.
According to the method for producing a protein fiber fabric according to the present embodiment, by using the protein fibers obtained by the production method according to the present invention as described above, a protein fiber fabric in which shrinkage at the time of first contact with moisture after production is more reliably suppressed and generation of crimp is also suppressed is easily produced.
The protein fiber used for the production of the protein fiber fabric may be a short fiber or a long fiber. In addition, such protein fibers may be used alone or in combination with other fibers. That is, when the protein fiber fabric is produced, a single yarn composed only of the protein fibers obtained by the production method according to the present invention and a composite yarn obtained by combining the protein fibers obtained by the production method according to the present invention and other fibers may be used singly or in combination thereof as material yarns. The other fibers refer to protein fibers obtained by a production method other than the production method according to the present invention, protein-free fibers, and the like. Moreover, the composite yarn includes, for example, a blended yarn, a combined filament yarn, a covering yarn, and the like.
The type of the protein fiber fabric produced according to the method for producing a protein fiber fabric according to the present embodiment is also not particularly limited. The protein fiber fabric may be, for example, a woven fabric or a knitted fabric, or a nonwoven fabric. Furthermore, the woven fabric may have, for example, a plain weave, a twill weave, a satin weave, or the like, and the type of yarn used may be one type or a plurality of types. The knitted fabric may be, for example, a warp knitted fabric such as tricot and raschel, a weft knitted fabric such as flat knitting and circular knitting, and the type of yarn to be used may be one type or a plurality of types.

Examples

Hereinafter, the present invention will be described more specifically based on Examples. However, the present invention is not limited to the following Examples.

[Test Example 1: Production of modified fibroin]

(1) Production of expression vector

Modified fibroin (PRT799) having the amino acid sequence set forth in SEQ ID NO: 15 was designed. A nucleic acid encoding the designed modified fibroin was synthesized. In the nucleic acid, an NdeI site was added to the 5' end and an EcoRI site was added downstream of the stop codon. The nucleic acid was cloned into a cloning vector (pUC118). Thereafter, the nucleic acid was enzymatically cleaved by treatment with NdeI and EcoRI, and then recombined into a protein expression vector pET-22b (+) to obtain an expression vector.

(2) Expression of protein

Escherichia coli BLR (DE3) was transformed with the obtained expression vector. The transformed Escherichia coli was cultured in 2 mL of an LB culture medium containing ampicillin for 15 hours. The culture solution was added to 100 mL of a seed culture medium containing ampicillin (Table 4) so that the OD600 reached 0.005. The temperature of the culture solution was maintained at 30°C, and flask culture was carried out (for about 15 hours) until the OD600 reached 5, thereby obtaining a seed culture solution.

[Table 4]

| Seed culture medium | |
|---|---|
| Reagent | Concentration (g/L) |
| Glucose | 5.0 |
| $KH_2PO_4$ | 4.0 |
| $K_2HPO_4$ | 9.3 |
| Yeast Extract | 6.0 |
| Ampicillin | 0.1 |

The seed culture medium was added to a jar fermenter to which a 500 mL production medium (Table 5) was added so that OD600 was 0.05. The culture was performed while maintaining the culture solution temperature at 37°C and constantly controlling the pH to 6.9. Further, the dissolved oxygen concentration in the culture solution was maintained at 20% of the dissolved oxygen saturation concentration.

[Table 5]

| Production medium | |
|---|---|
| Reagent | Concentration (g/L) |
| Glucose | 12.0 |
| $KH_2PO_4$ | 9.0 |
| $MgSO_4 \cdot 7H_2O$ | 2.4 |
| Yeast Extract | 15 |
| $FeSO_4 \cdot 7H_2O$ | 0.04 |
| $MnSO_4 \cdot 5H_2O$ | 0.04 |
| $CaCl_2 \cdot 2H_2O$ | 0.04 |
| Adecanol (Adeka, LG-295S) | 0.1 (mL/L) |

Immediately after glucose in the production medium was completely consumed, a feed solution (455 g/1 L of glucose, 120 g/1 L of Yeast Extract) was added at a rate of 1 mL/min. The culture was performed while maintaining the culture solution temperature at 37°C and constantly controlling the pH to 6.9. Further, the dissolved oxygen concentration in the culture solution was maintained at 20% of the dissolved oxygen saturation concentration, and the culture was performed for 20 hours. Thereafter, 1 M isopropyl-β-thiogalactopyranoside (IPTG) was added to the culture solution to a final concentration of 1 mM to induce the expression of the modified fibroin. The culture solution was centrifuged 20 hours after addition of IPTG, and bacterial cells were recovered. SDS-PAGE was conducted using the bacterial cells prepared from the culture solutions obtained before the addition of IPTG and after the addition of IPTG. The expression of the target modified fibroin which depended on the addition of IPTG was confirmed by the appearance of a band of the size of the target modified fibroin.

(3) Purification of protein

The bacterial cells recovered 2 hours after the addition of IPTG were washed with 20 mM Tris-HCl buffer solution (pH 7.4). The bacterial cells after washing were suspended in 20 mM Tris-HCl buffer (pH 7.4) containing about 1 mM PMSF, and the cells were disrupted with a highpressure homogenizer (manufactured by GEA Niro Soavi). The disrupted cells were centrifuged to obtain a precipitate. The obtained precipitate was washed with a 20 mM Tris-HCl buffer (pH 7.4) until the purity of the precipitate became high. The precipitate after washing was suspended in a 8 M guanidine buffer (8 M guanidine hydrochloride, 10 mM sodium dihydrogen phosphate, 20 mM NaCl, 1 mM Tris-HCl, pH 7.0) so that the

concentration of the precipitate was 100 mg/mL, and dissolved by stirring with a stirrer for 30 minutes at 60°C. After dissolution, dialysis was performed with water using a dialysis tube (cellulose tube 36/32, manufactured by Sanko Junyaku Co., Ltd.). A white aggregated protein obtained after the dialysis was collected by centrifugation, moisture was removed with a lyophilizer, and the lyophilized powder was collected, thereby obtaining the modified fibroin (PRT799).

[Test Example 2: Production and evaluation of protein fiber]

(1) Production of protein fibril

Dimethyl sulfoxide (DMSO) in which LiCl was dissolved so as to be 4.0 mass% was prepared as a solvent, and a lyophilized powder of the modified fibroin (PRT799) was added thereto so as to have a concentration of 24 mass%, and dissolved for 3 hours using a shaker. Thereafter, insoluble matters and foams were removed to obtain a modified fibroin solution (spinning dope).

In the case of using a wet shrinkage method as the pre-shrinking step (Example 1 and Comparative Example 2), a protein fibril was produced by a spinning apparatus according to a spinning apparatus 100 illustrated in Fig. 1. Specifically, the prepared spinning dope was filtrated with a metal filter having an opening of 5 μm at 60°C, subsequently allowed to stand in a 30 mL-stainless steel syringe to remove foams, and then discharged from a solid nozzle having a needle diameter of 0.2 mm into a 100 mass% methanol coagulation bath. The discharge temperature was 60°C. After coagulation, the discharged product was washed and then immersed in hot water at 90°C for 10 seconds to perform a pre-shrinking step. At this time, the protein fibril had a ratio of the take-up speed to the feed speed of less than 1 (0.6 times), which can be called a relaxed state. After drying at 100°C, the obtained original yarn was wound (winding step) to obtain a protein fibril (modified fibroin fibril).

In the case of using a dry shrinkage method as the pre-shrinking step (Example 2 and Comparative Example 3), a protein fibril was produced by a spinning apparatus according to a spinning apparatus 200 illustrated in Fig. 2. Specifically, the prepared spinning dope was filtrated with a metal filter having an opening of 5 μm at 60°C, subsequently allowed to stand in a 30 mL-stainless steel syringe to remove foams, and then discharged from a solid nozzle having a needle diameter of 0.2 mm into a 100 mass% methanol coagulation bath. The discharge temperature was 60°C. After coagulation, the discharged product was washed and dried, and heated at 240°C for 2 seconds to perform a pre-shrinking step. At this time, the protein fibril had a ratio of the take-up speed to the feed speed of less than 1 (0.5 times), which can be called a relaxed state. The obtained original yarn was wound (winding step) to obtain a protein fibril (modified fibroin fibril).

In the case where the pre-shrinking step was not performed (Comparative Example 1), a protein fibril was produced by a conventional dry-wet spinning apparatus (a device obtained by removing the water bath 46 from the spinning apparatus 100 illustrated in Fig. 1). Specifically, the prepared spinning dope was filtrated with a metal filter having an opening of 5 μm at 60°C, subsequently allowed to stand in a 30 mL-stainless steel syringe to remove foams, and then discharged from a solid nozzle having a needle diameter of 0.2 mm into a 100 mass% methanol coagulation bath. The discharge temperature was 60°C. After coagulation, the obtained original yarn was wound, and naturally dried to obtain a protein fibril (modified fibroin fibril).

In the case of using a wet shrinkage method as the post-shrinking step (Example 2 and Comparative Example 1), the post-shrinking step was performed using a device according to the devices illustrated in Figs. 6(a) and 6(b). Specifically, the protein fibril was pulled out from the wound product of the protein fibril to obtain a skein-like protein fibril (see Fig. 6(a)). Then, the skein-like protein fibril was immersed in hot water at 90°C for 15 minutes to perform the post-shrinking step. Thereafter, the obtained protein fibril was naturally dried to obtain a protein fiber (modified fibroin fiber).

In the case of using a dry shrinkage method as the post-shrinking step (Example 1), the post-shrinking step was performed using a device according to the devices illustrated in Figs. 6 (a) and 6 (c). Specifically, the protein fibril was pulled out from the wound product of the protein fibril to obtain a skein-like protein fibril (see Fig. 6(a)). Then, the skein-like protein fibril was brought into contact with a high temperature heater heated to 240°C and held for 10 seconds to perform the post-shrinking step. As a result, a protein fiber (modified fibroin fiber) was obtained.

(2) Evaluation of shrinkage rate of protein fiber

The modified fibroin fiber (test piece) of each of Examples and Comparative Examples cut to a length of 300 mm was immersed in water at 40°C or 90°C for 10 minutes without a load. Each test piece taken out of water was dried at room temperature for 2 hours. Thereafter, the length of the test piece (fiber length after drying) was measured, and the shrinkage rate was measured. The results are shown in Table 6. The shrinkage rate is a numerical value calculated by the following equation (1). The "fiber length before immersion" is 300 mm.

$$\text{Shrinkage rate} = (1 - \text{Fiber length after drying}/\text{Fiber length before immersion}) \times 100 \quad (1)$$

(3) Evaluation of crimp of protein fiber

The presence or absence of crimp of the test piece (test piece after drying) for which the shrinkage rate was measured in (2) was visually confirmed. The results are shown in Table 6.

[Table 6]

| | Pre-shrinking step | Post-shrinking step | Water temperature 40°C | | Water temperature 90°C | |
|---|---|---|---|---|---|---|
| | | | Shrinkage rate | Crimp | Shrinkage rate | Crimp |
| Example 1 | Wet | Dry | 2% | None | 7% | None |
| Example 2 | Dry | Wet | 0% | None | 3% | None |
| Comparative Example 1 | - | Wet | 0% | Occurred | 3% | Occurred |
| Comparative Example 2 | Wet | - | 3% | None | 15% | None |
| Comparative Example 3 | Dry | - | 1% | None | 12% | None |

In the protein fibers subjected to both the pre-shrinking step and the post-shrinking step (Example 1 and Example 2), shrinkage at the time of first contact with moisture after production was sufficiently suppressed, and crimp did not also occur. In the protein fiber subjected to only the post-shrinking step (Comparative Example 1), shrinkage at the time of first contact with moisture after production was suppressed, but crimp occurred. In the protein fibers subjected to only the pre-shrinking step (Comparative Example 2 and Comparative Example 3), although crimp did not occur, shrinkage at the time of first contact with moisture after production (in particular, shrinkage at the time of contact with moisture at high temperature) was large.

Reference Signs List

| | |
|---|---|
| 1 | Extrusion device |
| 4 | Drying device |
| 6 | Doping liquid |
| 20 | Coagulation bath |
| 21 | Washing bath |
| 36 | Protein fibril |
| 38 | Protein fiber |
| 42 | Feed roller |
| 44 | Winder |
| 46 | Water bath |
| 48 | Dryer |
| 54 | Heater |
| 56 | Presser roller |
| 58 | Hot roller |
| 60 | High temperature heating/relaxing device |
| 100, 200 | Spinning apparatus |
| 300, 400 | Post-shrinking device |

## Claims

**1.** A method for producing a protein fiber, the method comprising:

(i) drawing a protein fibril;
(ii) shrinking the protein fibril in a relaxed state after drawing and before being wound;
(iii) winding the protein fibril to obtain a wound protein fibril; and
(iv) shrinking the wound protein fibril in a relaxed state to produce a protein fibre in which moisture-induced shrinkage is reduced.

**2.** The production method according to claim 1, wherein step (iv) is:

(a) a wet shrinking step of bringing the wound protein fibril into contact with a liquid or vapor in a relaxed state to shrink the wound protein fibril, or
(b) a dry shrinking step of heating the wound protein fibril in a relaxed state to shrink the wound protein fibril.

**3.** The production method according to claim 2, wherein the wet shrinking step is a step of immersing the wound protein fibril in a liquid in a relaxed state to shrink the wound protein fibril.

**4.** The production method according to claim 2 or 3, wherein the liquid or vapor has polarity.

**5.** The production method according to claim 4, wherein the liquid is water, and the vapor is water vapor.

**6.** The production method according to any one of claims 1 to 5, wherein the protein fibril includes a structural protein.

**7.** The production method according to claim 6, wherein the structural protein is modified fibroin.

**8.** The production method according to claim 7, wherein the modified fibroin is modified spider silk fibroin.

**9.** A method for producing a protein fiber fabric, comprising a step of preparing a fabric using the protein fibers obtained by the production method according to any one of claims 1 to 8.

## Patentansprüche

**1.** Verfahren zum Produzieren einer Proteinfaser, wobei das Verfahren Folgendes umfasst:

(i) Ziehen einer Proteinfibrille;
(ii) Schrumpfen der Proteinfibrille in einem entspannten Zustand nach dem Ziehen und bevor sie gewickelt wird;
(iii) Wickeln der Proteinfibrille, um eine gewickelte Proteinfibrille zu erhalten; und
(iv) Schrumpfen der gewickelten Proteinfibrille in einem entspannten Zustand, um eine Proteinfaser zu produzieren, in der feuchtigkeitsinduzierte Schrumpfung reduziert ist.

**2.** Produktionsverfahren nach Anspruch 1, wobei Schritt (iv) wie folgt ist:

(a) ein Nassschrumpfungsschritt des Bringens der gewickelten Proteinfibrille in Kontakt mit einer Flüssigkeit oder einem Dampf in einem entspannten Zustand, um die gewickelte Proteinfibrille zu schrumpfen, oder
(b) ein Trockenschrumpfungsschritt des Erhitzens der gewickelten Proteinfibrille in einem entspannten Zustand, um die gewickelte Proteinfibrille zu schrumpfen.

**3.** Produktionsverfahren nach Anspruch 2, wobei der Nassschrumpfungsschritt ein Schritt des Eintauchens der gewickelten Proteinfibrille in einer Flüssigkeit in einem entspannten Zustand ist, um die gewickelte Proteinfibrille zu schrumpfen.

**4.** Produktionsverfahren nach Anspruch 2 oder 3, wobei die Flüssigkeit oder der Dampf Polarität aufweist.

**5.** Produktionsverfahren nach Anspruch 4, wobei die Flüssigkeit Wasser ist und der Dampf Wasserdampf ist.

**6.** Produktionsverfahren nach einem der Ansprüche 1 bis 5, wobei die Proteinfibrille ein Strukturprotein beinhaltet.

**7.** Produktionsverfahren nach Anspruch 6, wobei das Strukturprotein modifiziertes Fibroin ist.

**8.** Produktionsverfahren nach Anspruch 7, wobei das modifizierte Fibroin modifiziertes Spinnenseidenfibroin ist.

**9.** Verfahren zum Produzieren eines Proteinfasergewebes, umfassend einen Schritt des Vorbereitens eines Gewebes unter Verwendung der Proteinfasern, die durch das Produktionsverfahren nach einem der Ansprüche 1 bis 8 erhalten werden.

## Revendications

**1.** Procédé permettant la production d'une fibre protéique, le procédé comprenant :

(i) l'étirage d'une fibrille protéique ;
(ii) le rétrécissement de la fibrille protéique dans un état relâché après l'étirage et avant d'être enroulée ;
(iii) l'enroulement de la fibrille protéique pour obtenir une fibrille protéique enroulée ; et
(iv) le rétrécissement de la fibrille protéique enroulée dans un état relâché pour produire une fibre protéique dans laquelle le rétrécissement induit par l'humidité est réduit.

**2.** Procédé de production selon la revendication 1, ladite étape (iv) étant :

(a) une étape de rétrécissement par voie humide consistant à la mise en contact de la fibrille protéique enroulée avec un liquide ou une vapeur dans un état relâché pour rétrécir la fibrille protéique enroulée, ou
(b) une étape de rétrécissement à sec consistant au chauffage de la fibrille protéique enroulée dans un état relâché pour rétrécir la fibrille protéique enroulée.

**3.** Procédé de production selon la revendication 2, ladite étape de rétrécissement par voie humide étant une étape d'immersion de la fibrille protéique enroulée dans un liquide dans un état relâché pour rétrécir la fibrille protéique enroulée.

**4.** Procédé de production selon la revendication 2 ou 3, ledit liquide ou ladite vapeur possédant une polarité.

**5.** Procédé de production selon la revendication 4, ledit liquide étant de l'eau et ladite vapeur étant de la vapeur d'eau.

**6.** Procédé de production selon l'une quelconque des revendications 1 à 5, ladite fibrille protéique comprenant une protéine de structure.

**7.** Procédé de production selon la revendication 6, ladite protéine de structure étant la fibroïne modifiée.

**8.** Procédé de production selon la revendication 7, ladite fibroïne modifiée étant une fibroïne de soie d'araignée modifiée.

**9.** Procédé permettant la production d'un tissu de fibres protéiques, comprenant une étape de préparation d'un tissu en utilisant les fibres protéiques obtenues par le procédé de production selon l'une quelconque des revendications 1 à 8.

FIG. 1
100
5
36
17
22
4
52
46
47
56
54
50
18g
18f
18e
14
12
3
21
13
18d
18c
18b
11
2
20
19
9
18a
7
6
8
1

200
5
36
63
62
61
60
17
32
31
4
25
30
18e
14
12
3
21
13
18d
18c
18b
11
2
20
19
9
7
6
18a
8
1

FIG. 2

FIG. 3
67
TEMPERATURE CONTROLLER
66
SPEED CONTROLLER
36
63
62
60
64
61
17
32
4
31

300
44
38
58
58
48
52
47
56
46
54
50
36
42

FIG. 4

FIG. 5
400
67
TEMPERATURE CONTROLLER
60
64
63
36
38
42
50
61
62
66
44
SPEED CONTROLLER

*FIG. 6*

(a)

36
71
42
70

(b)

71
70
46
47
54

(c)

71
70
64

FIG. 7
AAAA
50AA
AAAA
100AA
AAAA
10AA
AAAA
20AA
AAAA
30AA
AAAA
PATTERN 1
PATTERN 2
PATTERN 3
PATTERN 4
.....

FREQUENCY
3.5
3
2.5
2
1.5
1
0.5
0
z/w(%)
0
5
10
15
20
25
30
35
40
45
50
55
60
65
70
75
80
85
90
95
100

FIG. 8

FREQUENCY
0
5
10
15
20
25
30
35
40
0 4 8 12 16 20 24 28 32 36 40 44 48 52 56 60 64 68 72 76 80 84 88 92 96 100
x/y (%)

FIG. 9

AAAA
30
4
4
AAAA
20
4
AAAA
10
AAAA
40
4
4
AAAA
50
4
4
AAAA

FIG. 10

FIG. 11

REGION A

AAAA
50
GPGXX GPGXX
AAAA
40
GPGXX GPGXX
AAAA
10
AAAA
20
GPGXX
AAAA
30
GPGXX GPGXX
AAAA

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018164020 A
- WO 2019066047 A1


### Non-patent literature cited in the description


- *Nucleic Acid Res.*, 1982, vol. 10, 6487
- *Methods in Enzymology*, 1983, vol. 100, 448
- **KYTE J** ; **DOOLITTLE R**. A simple method for displaying the hydropathic character of a protein. *J. Mol. Biol.*, 1982, vol. 157, 105-132